# EUROPEAN PATENT SPECIFICATION

(11) **EP 0 799 050 B1**
(45) Date of publication and mention of the grant of the patent: **11.08.2004**
(21) Application number: 95942562.0
(22) Date of filing: 06.12.1995
(51) Int. Cl.: A61K 51/08, C07K 14/47

(54) **RADIOLABELED ANNEXIN-GALACTOSE CLUSTER CONJUGATES**
RADIOAKTIVMARKIERTE ANNEXIN-GALAKTOSE-CLUSTER KONJUGATE
CONJUGUES GROUPES ANNEXINE-GALACTOSE RADIOMARQUES

(30) Priority: 07.12.1994 US 351653; 23.01.1995 WO PCT/US95/00953; 13.06.1995 WO PCT/US95/07599
(43) Date of publication of application: 08.10.1997
(73) Proprietor: NEORX CORPORATION, Seattle Washington 98119-4114 (US)
(72) Inventor: THEODORE, Louis J., Lynnwood, WA 98037 (US); KASINA, Sudhakar, Mercer Island, WA 98040 (US); RENO, John M., Brier, WA 98036 (US)
(74) Representative: Gowshall, Jonathan Vallance
(86) International application number: PCT/US1995/015851
(87) International publication number: WO 1996/017618

(56) References cited:
- WO-A-91/07187
- WO-A-91/09628
- WO-A-92/19279
- WO-A-94/19024
- WO-A-95/34315
- US-A- 4 897 255
- US-A- 5 258 497
- US-A- 5 371 184
- US-A- 5 627 036
- HASHIDA M ET AL: "Hepatic targeting of drugs and proteins by chemical modification" JOURNAL OF CONTROLLED RELEASE, vol. 1, no. 36, September 1995, page 99-107 XP004037472
- PRESCOTT J H ET AL: "Larch arabinogalactan for hepatic drug delivery: isolation and characterization of a 9 kDa arabinogalactan fragment" CARBOHYDRATE RESEARCH, vol. 1, no. 278, 30 November 1995, page 113-128 XP004018867
- STRATTON JR ET AL: "Selective uptake of radiolabeled annexin V on acute porcine left atrial thrombi." CIRCULATION, NOV 15 1995, 92 (10) P3113-21, UNITED STATES, XP002058828
- KASINA S ET AL: "Preformed chelate Tc-99m radiolabeling of r-annexin V for arterial thrombus imaging" JOURNAL OF NUCLEAR MEDICINE, 37 (5 SUPPL.). 1996. 29P., XP002058829
- BRITISH JOURNAL OF CANCER, Volume 61, issued 1990, S.K. SHARMA et al., "Inactivation and Clearance of an Anti-CEA Carboxypeptidase G2 Conjugate in Blood After Localisation in a Xenograft Model", pages 659-662.
- JOURNAL OF NATIONAL CANCER INSTITUTE, Volume 79, Number 4, issued 04 October 1987, M.J. MATTES, "Biodistribution of Antibodies After Intraperitoneal or Intravenous Injection and Effect of Carbohydrate Modifications", pages 855-863.
- JOURNAL OF NUCLEAR MEDICINE AND ALLIED SCIENCES, Volume 32, Number 2, issued 1988, G. GALLI et al., "A Radiopharmaceutial for the Study of the Liver: 99mTc-DTPA-Asialo-Orosomucoid", pages 110-116.
- GLYCOCONJUGATE JOURNAL, Volume 4, issued 1987, R.T. LEE et al., "Preparation of Cluster Glycosides of N-Acetylgalactosamine That Have Subnanomolar Binding Constants Towards the Mammalian Hepatic GA1/Ga1NAc-specific Receptor", pages 317-328.
- BIOCHEMISTRY, Volume 15, Number 18, issued 1976, M.J. KRANTZ et al., "Attachment of Thioglycosides to Proteins: Enhancement of Liver Membrane Binding", pages 3963-3968.
- BIOCHEMISTRY, Volume 15, Number 18, issued 1976, Y.C. LEE et al., "2-Imino-2-Methoxyethyl 1-Thioglycosides: New Reagents for Attaching Sugars to Proteins", pages 3956-3962.

## Description

### Technical Field

The present invention is directed to radiolabeled annexin-galactose cluster conjugates. Also contemplated by the present invention are imaging protocols which involve the administration of a radiolabeled annexin-galactose cluster conjugate. The annexin component of the conjugate serves to deliver the radiolabeled active component of the conjugate to vascular thrombi target sites. The galactose cluster component of the conjugate facilitates rapid elimination of the radiolabeled annexin-galactose cluster conjugate from the circulation of a recipient.

### Background of the Invention

When patients present with chest pain, palpitations or any other symptoms of coronary trauma or disease, the presence of vascular thrombi in the heart is a potential significant complicating factor for treatment. If a medical practitioner could non-invasively determine whether one or more vascular thrombi were present and, if present, the location of those vascular thrombi, better evaluation of treatment options would be possible. Furthermore, if a medical practitioner could determine that no vascular thrombi were present, thereby eliminating a potential complication in treatment, cardiac conditions could be treated more safely and effectively.

Most present techniques for determining the presence of vascular thrombi are invasive and/or cumbersome, and/or fail to detect such thrombi with good sensitivity and specificity. Thus, an imaging agent useful for non-invasive vascular thrombi imaging is desirable.

Annexins are a class of proteins that are characterized by calcium-mediated binding to anionic phospholipids. Anionic phospholipids are about 20-fold more highly associated with activated platelets than quiescent platelets, and activated platelets are associated with vascular thrombi.

Iodinated annexin V has been shown to localize to vascular thrombi in vivo, but has suboptimal imaging characteristics, possibly due to the pronounced beta phase of blood clearance owing to possible transiodination and/or metabolic degradation with reincorporation into serum macromolecules or non-target tissues. Free radioactive iodine or iodine-containing metabolic degradation products exposed non-target tissues, especially the thyroid gland, to , radioactivity. In addition, the iodine radiolabel used is difficult to obtain and is not therefore practical for wide spread use. Consequently, improved radiolabeled annexin compounds are desirable.

In addition, conventional imaging and therapy are often plagued by the problem that the generally attainable targeting ratio (ratio of administered dose localizing to target versus administered dose circulating in blood or ratio of administered dose localizing to target versus administered dose migrating to bone marrow) is low. Improvement in targeting ratio is also sought.

### Summary of the Invention

The present invention provides an annexin conjugate suitable for radiolabeling with a diagnostic imaging agent comprising:
an annexin;
an N₂S₂ or N₃S compound capable of complexing a radionuclide, wherein the compound is associated with the annexin; and
a cluster of hexose or hexose based residues connected in a branched configuration, wherein the residues of said cluster are configured to be recognised by a liver receptor.
Preferred annexin-containing conjugates of the present invention are those suitable for radiolabeling with a diagnostic imaging agent including:
an annexin;
a cluster of galactose residues; and
an N₂S₂ chelate associated with the annexin.

Also provided by the present invention are radiolabeled annexin-galactose cluster conjugates suitable for imaging vascular thrombi, which radiolabeled annexin-galactose cluster conjugates include:
an annexin;
a cluster of galactose residues;
an N₂S₂ chelate associated with the annexin; and
a diagnostic radionuclide complexed by the chelate.

A preferred annexin for use in the present invention is annexin V. Preferred galactose clusters for use in the present invention incorporate a multiple of 4 galactoses. Other preferred galactose clusters for use in the present invention incorporate a multiple of 3 galactoses.

The galactose cluster component may bound to the annexin component and the chelate component via a trifunctional linker, such as lysine. An extender may be employed between the galactose cluster and the trifunctional linker to promote bioavailability of the galactose cluster. This embodiment of the present invention is favored if the chelate is characterized by a single functional group available and suitable for conjugation.

If the chelate component is characterized by greater than one functional group available and suitable for conjugation to other conjugate components, a structure such as the following is preferably employed: galactose cluster - bifunctional linker - chelate - bifunctional linker - annexin.

Preferred diagnostic radionuclides for use in the practice of the present invention are Tc-99m, Re-186 and Re-188, with Tc-99m being especially preferred. Vascular thrombi located in or near the heart are especially amenable to imaging in accordance with the present invention.

### Brief Description of the Drawings

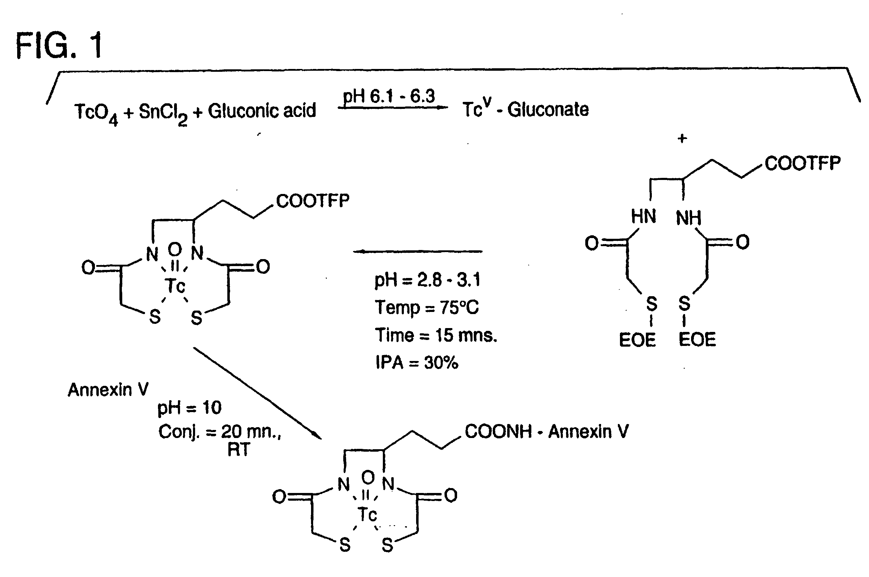

Fig. 1 schematically represents a method of radiolabeling annexin V.
Fig. 2 shows the blood clearance of Tc-99m-annexin V (o) and of Tc-99m-annexin V-galactose (Δ)
Fig. 3 schematically shows the synthesis of N,N'-bis(2-disulfidyl-4-carbonylphenyl)-1,3-propyldiamine.
Fig. 4 schematically shows the synthesis of N,N'-bis(disulfidyl-4-methylphenyl)-gamma,gamma'-diamino-isovalerate N-hydroxysuccinimide.
Figs. 5a and 5b schematically show the synthesis of an eight galactose-containing galactose cluster.
Fig. 6 schematically shows the synthesis of an extended eight galactose-containing galactose cluster.

### Detailed Description of the Invention

Prior to setting forth the invention, it may be helpful to set forth definitions of certain terms to be used within the disclosure.

Annexin: A class of compounds characterized by the ability to bind with high affinity to membrane lipids in the presence of millimolar concentrations of calcium. Annexins have been shown to exhibit anti-coagulatory effects that are mediated by the binding of annexins to negatively charged surface phospholipids (e.g., on activated platelets). This annexin-phospholipid binding is believed to block the activation of clotting factors by such negatively charged surface phospholipids. Prior to the recognition of the annexin class of molecules, members thereof were also referred to in the literature as placental anticoagulant proteins (e.g., PAP-1, 2, 3 and 4), lipocortins, calpactins, vascular coagulant (alpha and beta), calphobindin I, placental protein 4 (PP4), endonexin II, anchorin CII, calcium-dependent phospholipid binding protein, and the like. See Crumpton et al., Nature 345:212, 1990. Annexin-V is a prototypical annexin molecule used in the description of the present invention.

N₂S₂ Chelates: Diamide, dimercaptide bifunctional chelators of the NₓS_{y} family capable of stably complexing a radionuclide through two nitrogen atoms and two sulfur atoms that are appropriately positioned. N₂S₂ chelates are described in U.S. Patent No. 4,897,225, for example. Preferred chelates of this type include chelates having the following biphenyl backbone:

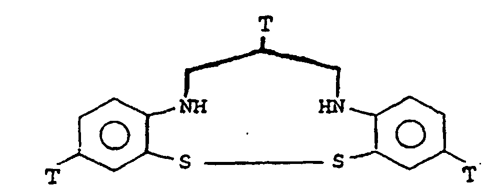

N₃S Chelates: Triamide, mercaptide bifunctional chelators of the NₓS_{y} family capable of stably complexing a radionuclide through three nitrogen atoms and one sulfur atom that are appropriately positioned. Preferred N₃S chelates are described in U.S. Patent No. 4,965,392, for example.

Radiolabeled Annexin: An annexin conjugated to a chelate having a radionuclide complexed therein.

Radiolabeled Annexin-Galactose: A galactose-derivatized annexin conjugated to a chelate having a radionuclide complexed therein.

Sugar cluster: A construct having a plurality of sugar residues configured to be recognized by a liver receptor. Such clusters are preferably constructed of sugar residues connected in a branched configuration, and are attached to other components of a sugar cluster-containing conjugate via a single point of attachment. Preferably, the branching network consists of two or three pronged branches, i.e., consists of 2, 4, 8, 16, 32 or 64 sugar residues or consists of 3, 9, 27, or 81 sugar residues.

Galactose cluster: A construct having from 3 to 64 galactose residues connected in a branched configuration. Preferably, the branching network consists of two or three pronged branches, i.e., consists of 2, 4, 8, 16, 32 or 64 galactose residues or consists of 3, 9, 27, or 81 galactose residues.

Radiolabeled Annexin-Galactose Cluster: A galactose cluster-derivatized annexin conjugated to a chelate having a radionuclide complexed therein.

Conjugate: A conjugate encompasses chemical conjugates (covalently or non-covalently bound), fusion proteins.

The present invention is directed to annexin-containing conjugates, radiolabeled annexin-galactose cluster conjugates and the use thereof for diagnostic imaging purposes. Radiolabeled annexins are characterized by the following: rapid accretion to target cell sites characterized by anionic phospholipids; short circulating half-life; in vivo stability against metabolic degradation or radionuclide separation from chelate; and amenability to packaging in cold kit format.

Radiolabeled annexin-galactose conjugates also exhibit the aforementioned characteristics, albeit to different degrees. For example, radiolabeled annexin-galactose conjugates exhibit a shorter circulating half-life than their radiolabeled annexin counterparts. Also, radiolabeled annexin-galactose , conjugates generally exhibit a lower binding affinity for target sites than their radiolabeled annexin counterparts. Successful diagnostic imaging involves both target site accumulation of signal and rapid elimination of non-targeted signal. Consequently, the enhanced elimination from a recipient's circulation of the radiolabeled-annexin-galactose conjugates provides a distinct opportunity to achieve diagnostic images in a shorter time frame.

Moreover, target site signal diminishes over time as a result of radioactive decay. In addition, metabolism of the target associated material also diminishes target signal. Consequently, shorter time frame imaging enhances the target:non-target ratio and total target signal, thereby garnering improved diagnostic information. ,

Radiolabeled annexin-galactose cluster conjugates of the present invention combine desirable features of radiolabeled annexins and radiolabeled annexin-galactose conjugates. For example, radiolabeled annexin-galactose cluster conjugates exhibit a shorter circulating half-life than their radiolabeled annexin counterparts. Also, radiolabeled annexin-galactose cluster conjugates generally exhibit a higher binding affinity for target sites than their radiolabeled annexin-galactose conjugate counterparts. Consequently, the enhanced elimination from a recipient's circulation and the substantial binding affinity maintenance of the radiolabeled-annexin-galactose cluster conjugates of the present invention invention offer the opportunity to achieve clear diagnostic images in a shorter time frame.

An embodiment of the present invention is directed to annexin-containing conjugates suitable for radiolabeling with a diagnostic imaging agent including:
an annexin;
a cluster of galactose residues; and
an N₂S₂ or N₃S chelate associated with the annexin.
Radiolabeled annexin-galactose cluster conjugates suitable for imaging vascular thrombi anywhere in the recipient (but particularly in or near the heart) are also contemplated, which radiolabeled annexin-galactose cluster conjugates incorporate an annexin, a cluster of galactose residues, an N₂S₂ or N₃S chelate, and further a diagnostic radionuclide complexed by the ' chelate.

A preferred embodiment of the present invention involves annexin-containing conjugates suitable for radiolabeling with a diagnostic imaging agent including:
an annexin;
a cluster of galactose residues; and
an N₂S₂ chelate associated with the annexin.
Radiolabeled annexin-galactose cluster conjugates suitable for imaging vascular thrombi are also contemplated, which radiolabeled annexin-galactose conjugates incorporate an annexin, a cluster of galactose residues, an N₂S₂ chelate, and further a diagnostic radionuclide complexed by the chelate.

For the visualization of vascular thrombi associated with a number of pathological conditions, a conjugate of an annexin with a chelate complexed with an imaging radionuclide, such as Tc-99m for example, is administered to a recipient for whom such a diagnosis is desired. The annexin portion of the conjugate localizes rapidly to target sites characterized by negatively charged surface phospholipids, such as vascular thrombi. The radionuclide is selected for its ability to be visualized via one of the various techniques therefor, e.g., gamma camera imaging. Because of the rapid accretion of annexins to the target site and the short serum half-life (generally less than 30 minutes) of annexins (which is not significantly lengthened upon radiolabeling), imaging of those target sites proceeds with little exposure of non-target sites to radioactivity.

Diagnostic imaging is dependent on signal-to-noise ratio. Improvements involving either target signal accumulation or noise reduction will enhance the efficacy of the diagnostic imaging product. For targeted imaging, noise reduction is synonymous with reducing background radioactivity, particularly blood pool activity. Annexins, including annexin V, are rapidly removed by the liver; however, only a fraction of the activity is removed with each pass of the circulating conjugate through the liver.

To more efficiently remove the background activity from the blood, an improvement in liver extraction of annexin-containing conjugates could be employed. A preferred method to enhance liver extraction involves derivatizing the annexin-containing conjugate with a moiety, such as galactose, that is recognized by a liver receptor. The efficiency of liver receptor extraction of the moiety recognized thereby will result in increased "per pass" removal by the liver of derivatized annexin-containing conjugate (e.g., annexin-galactose conjugate) in comparison to the amount of underivatized annexin-containing conjugate so removed.

Further improvement in signal-to-noise ratio can be achieved by derivatizing the annexin-containing conjugate with a cluster of galactose molecules recognized by a liver receptor. The efficiency of liver receptor extraction of the moiety recognized thereby will result in increased "per pass" removal by the liver of derivatized annexin-containing conjugate (e.g., annexin-galactose cluster conjugate) in comparison to the amount of underivatized annexin-containing conjugate so removed. In addition, the multiple galactose residues arranged in a cluster are bound to the annexin conjugate component via a single point of attachment. Consequently, less or no reduction in annexin target binding affinity resulting from galactose derivatization may be achieved in comparison to annexin-galactose conjugates.

Annexins are generally (with the most notable exception being annexin II) single chain, non-glycosylated proteins of approximately 33-72 kilodalton molecular weight. Annexins possess a number of biological activities associated with calcium ion-mediated binding.

Investigations have shown that annexins bind with high affinity to anionic membrane lipids in the presence of millimolar concentrations of calcium. In the presence of calcium, these proteins have an especially high affinity for negatively charged phospholipids, such as phosphatidylserine, phosphatidylglycerol, phosphatidic acid, or phosphatidylinositol. See, for example, Funakoshi et al., Biochem. 26: 5572-78, 1987; and Tait et al., Biochem. 27: 6268-76, 1988. Such negatively charged phospholipids are associated with vascular thrombi (e.g., are located on the surface of activated human platelets).

Annexins exert anti-coagulatory effects. Coagulation inhibition is mediated by the binding of annexins to negatively charged surface phospholipids (e.g., present on the surface of activated platelets). This binding is believed to block the activation of clotting factors by such negatively charged surface phospholipids. Annexins localize to target sites bearing anionic phospholipids rapidly, i.e., in a matter of approximately 5 to 30 minutes depending on circulating levels thereof, but remain circulating in the serum for a somewhat longer time period (circulating half-life < 30 minutes). Example III below discusses results of imaging experiments wherein vascular thrombi were visualized in planar images at an average time (following annexin administration);of 82 minutes.

Because of these properties, annexins or annexins conjugated to diagnostic or therapeutic agents may be employed in protocols for the diagnosis or treatment of vascular thrombi associated with a number of indications, such as DVT (deep vein thrombosis), PE (pulmonary embolism), myocardial infarction, atrial fibrillation, problems with prosthetic cardiovascular materials and stroke. Exemplary diagnostic protocols and experimentation employing radiolabeled annexins are set forth below to further elucidate this aspect of the present invention.

An example of a preferred annexin useful in the practice of the present invention is Annexin V, which was isolated by Bohn in 1979 from human placenta, a rich source of annexins, and termed Placenta Protein 4 (PP4). Annexin V has been expressed in E. coli.
Also, a full length cDNA clone of annexin V has been obtained and subcloned in expression vectors, thereby facilitating the production of fusion proteins containing annexin V. Annexin V consists of four domains (four tandem, imperfect repeats of about 75 amino acid residues, Funakoshi et al., Biochem. 26: 8087-92, 1987), wherein each domain is made up of 5 alpha helices. From the side, the annexin V molecule appears crown-like with at least four calcium binding sites on its convex surface, through which annexin-phospholipid interactions are mediated. Other annexin molecules are also useful in the practice of the present invention, and the discussions relating to annexin V herein apply generally to annexin molecules.

Because annexin V has a plurality of calcium binding sites, and because annexin V binding to negatively charged phospholipids is mediated by calcium, an engineered molecule consisting of one or more individual annexin V domains may be employed in imaging protocols of the present invention. Also, the annexin molecule may be partitioned at a position or positions different from the domain boundaries to provide an engineered molecule capable of calcium-mediated binding of anionic phospholipids. Also, annexin V may be altered at one or more amino acid residues, so long as the affinity of annexin V for anionic phospholipids is not significantly impaired. The degree of annexin binding to phospholipids may be quantified by fluorescence quenching as described by Tait et al., J. Biol. Chem. 264: 7944-49, 1989.

Among annexins, annexin V has the strongest binding affinity (K_{d} < 10⁻¹⁰ M) for phospholipid vesicles containing 80% phosphatidylcholine and 20% phosphatidylserine under conditions comparable ,to plasma and extracellular fluid (1.2 mM ionized calcium, 0.15 M ionic strength). This binding is reversible and calcium dependent.

To decrease the background radiolabel activity, annexins may be derivatized with hexose or hexose-based moieties. More specifically, annexins may be derivatized to incorporate one or more hexoses (six carbon sugar moieties) recognized by Ashwell receptors or other liver receptors, such as the mannose/N-acetylglucosamine receptor, which are associated with endothelial cells and/or Kupffer cells of the liver, or by the mannose 6-phosphate receptor. Exemplary of such hexoses and hexose-based moieties are galactose, mannose, mannose 6-phosphate, *N*-acetylglucosamine and pentamannosyl phosphate. Other moieties recognized by Ashwell receptors, including glucose, N-galactosamine, N-acetylgalactosamine, thioglycosides of galactose and, generally, D-galactosides and glucosides , may also be used in the practice of the present invention.

Galactose is the prototypical hexose employed for the purposes of this description. Galactose thioglycoside conjugation to a protein is preferably accomplished in accordance with the teachings of Lee et al., "2-Imino-2-methoxyethyl 1-Thioglycosides: New Reagents for Attaching Sugars to Proteins," Biochemistry 15(18): 3956, 1976. Another useful galactose thioglycoside conjugation method is set forth in Drantz et al, "Attachment of Thioglycosides to Proteins: Enhancement of Liver Membrane Binding," Biochemistry 15(18): 3963, 1976. Annexin-galactose conjugation is also discussed in the Examples set forth herein.

Hexose conjugation to the annexin via chemical methods can occur either prior to (post-formed approach) or following (pre-formed approach) conjugation of the chelate to the annexin. Hexose chemical conjugation is preferably conducted prior to chelate conjugation, however.

The number of galactose residues on the product conjugates will range from 1 to the maximum number of galactoses that do not significantly diminish the binding affinity of annexin for its target. For example, galactose derivatization that preserves at least 20% of native annexin binding activity is preferred, with the preservation of at least 50% of native annexin binding activity more preferred. The theoretically possible maximum number of galactose residues located on the annexin molecule is 22 (i.e., the number of lysine residues within the annexin structure). An exemplary number of galactose residues on radiolabeled annexin-galactose conjugates of the present invention ranges between 1 and 5.

Hexose clusters are preferably employed in the practice of the present invention. Galactose clusters are the prototypical hexose clusters employed for the purposes of this description. Design of hexose clusters of the present invention is conducted with the following criteria in mind, as set forth in the context of the design of a galactose cluster:
1) Number of Galactoses in a Cluster;
2) Distance Between Galactoses in the Cluster; and
3) Distance Between Galactose Cluster and the Annexin Conjugate Component.

With regard to criteria number 1, literature indicates that galactose receptors on the surface of human hepatocytes are grouped as heterotrimers and, perhaps, bis-heterotrimers. See, for example, Hardy. et al., Biochemistry, 24: 22-8, 1985. For optimal affinity to such receptors, the present inventors believe that each galactose cluster should preferably contain at least three galactose residues. In general, the greater the number of sugars in a cluster, the greater the propensity for the cluster to be recognized by liver receptors.

Increased sugar cluster size may impair annexin binding to target. If significant impairment in annexin binding to target (e.g.. reduction to < 20% of native annexin binding) is observed, a longer linker between the two moieties may be used or such large clusters should not be employed in radiolabeled annexin-galactose cluster conjugates of the present invention.

With respect to criteria number 2, the galactose receptors within each trimer are separated from each other by distances of 1.5, 2.2 and 2.5 nm (15, 22 and 25 angstroms). Consequently, the present inventors believe that the galactoses within a cluster should preferably be.. separated by flexible linkers allowing separation of at least 2.5 nm (25 angstroms). The spacing between sugar residues is likely to be more important if the number of sugar residues is small. With larger constructs, appropriate spacing is likely to occur with respect to sugar residues that are not immediate neighbors (i.e., sugar residues that are farther apart than those that are immediate neighbor). Assuming an average bond length of 150 pm (1.5 angstroms), preferred sugar clusters of the present invention are characterized by separation of neighboring sugar residues by 10 bond lengths or more. Other preferred constructs involve galactose clusters characterized by separation of neighboring sugar residues by 25 bond lengths or more.

Regarding criteria number 3, the distance between the annexin and the galactose cluster should be sufficient to obviate any adverse steric effects upon annexin binding to target caused by the size or orientation of the galactose cluster. This distance is preferably greater than 7 bond lengths or 1 nm (10 angstroms). If necessary, an extender molecule is incorporated between the galactose cluster and the linker (which joins the galactose cluster and the annexin component) or between the annexin and the linker to provide the requisite distance.

While the foregoing parameters appear to be optimal for galactose, it should be noted that these factors may vary with other hexoses or mixtures thereof, which may or may not bind to the same receptors, or may bind differently. Given the teachings in this application, one skilled in the art can, using available synthesis techniques, attach an annexin and an active agent to other hexose clusters and identify those constructs which provide optimal performance.

Any branched sugar structures that meet the criteria described above may be employed in the practice of the present invention. Preferred galactose clusters of the present invention are. of the following structures: 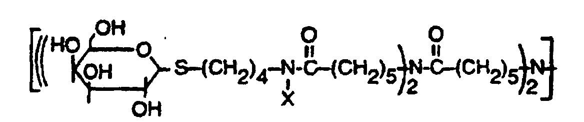 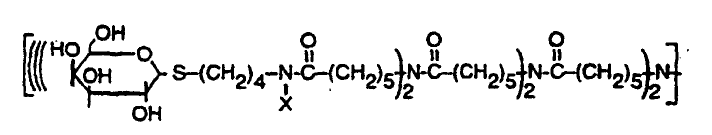 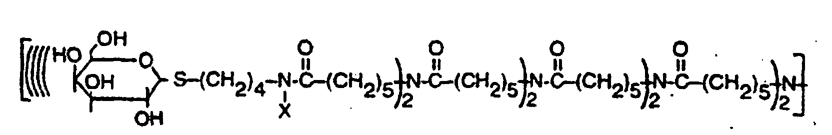 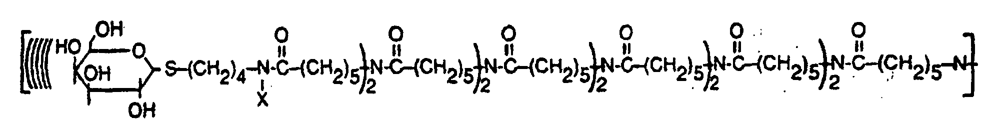 wherein X is preferably H or methyl, resulting in galactose clusters bearing 4, 8, 16 and 32 galactose residues, respectively. Further iteration in the branching scheme allows expansion of the galactose cluster to include 32, 64, etc. galactose residues. In addition, the linker moiety between the sugar itself and the branching structure (shown as -S-(CH₂)₄-NX-) may be variable in length.

Alternative branching structures may also be employed in the design of galactose clusters in accordance with the present invention. For example, other constructs wherein the branching results in a doubling of the number of galactose residues may be employed. In addition, constructs wherein branching results in a tripling or other convenient multiplying of the number of galactose residues are also contemplated by the present invention.

Another potential branching construction is based upon the molecule bis-homotris: (HO-CH₂)₃-C-NH₂. The sulfhydryl-containing derivative of this molecule may also be used. In this embodiment of the present invention, each arm of the bis-homotris molecule is extended and terminated in a carboxylic acid: [HO₂C-(CH₂)_{y}-Z-CH₂]₃-C-NH₂, where Z is S or O and y ranges from 1 to 10. For this embodiment of the present invention, a preferred galactose cluster is characterized by the following structures: 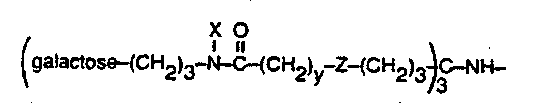 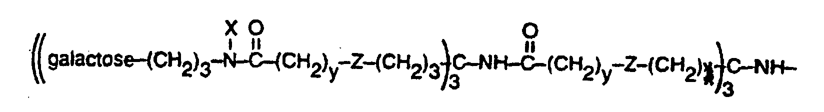 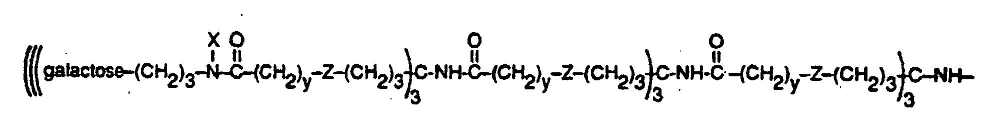 wherein X is preferably H or methyl; y ranges from 1 to 10; and Z is O or S. The above structures bear 3, 9 and 27 galactose residues, respectively. Further iteration of the branching allows expansion to include 81, etc. galactose residues.

In embodiments of the present invention wherein the chelate is characterized by a single functional group available and suitable for conjugation, the annexin, chelate and galactose cluster components are preferably joined via a trifunctional linker. Functional groups that are "available" for conjugation are those that are not prevented by steric constraints from conjugate formation. Functional groups that are "suitable" for conjugation are those that are capable, in a chemical sense, of reacting with available functional groups associated with other conjugate components. In addition, conjugation of "suitable" functional groups does not substantially impair a, necessary function of the component with which the functional group is associated. For example, a functional group located in the complementarity determining region of an antibody targeting moiety will generally not be "suitable" for conjugation, because the targeting ability of the antibody is likely to be substantially impaired by such binding.

Useful trifunctional linkers are amenable to binding with functional groups available on,the three conjugate components or any extender moieties employed. in conjugate construction. A useful trifunctional linker is lysine, wherein the alpha-amino, epsilon-amino and carboxyl functional groups are used. One skilled in art could identify other trifunctional linkers and use the same in the practice of the present invention as set forth herein.

Extender molecules useful in the present invention are bifunctional moieties capable of binding with either the annexin component and the linker or the galactose cluster component and the linker. Suitable extender molecules include aminocaproate moieties, HS-(CH₂)ₙCOOH or an activated ester form thereof wherein n ranges from 2 to 5. One of ordinary skill in the art is capable of identifying and using other suitable extender molecules as described herein. Alternatively, the extender function can be served by an appropriately constructed linker.

Also, binding facilitation moieties may also be employed in the present invention. Such moieties are bifunctional and facilitate binding the conjugate components, e.g., galactose cluster, annexin, chelate, linker, and extender. Examples of such binding facilitation moieties include urea functionalities, thiourea functionalities, succinate bridges and maleimides. Such binding facilitation moieties are amenable to identification and use by those skilled in the art.

An example of a linker-extender-binder facilitation system is shown below: 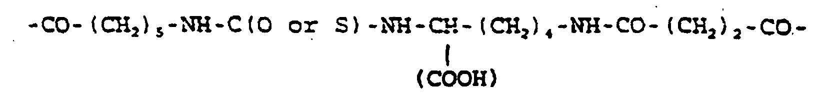 wherein the alpha-amine of the lysine linker is bound via a urea or thiourea functionality to an amino caproate spacer (which, in turn, binds to a galactose cluster that is not shown); the lysine carboxylate is available for linkage to a chelate (not shown); and the epsilon-amine of the lysine linker is available for linkage to a lysine residue of the annexin component (not shown) via a succinate bridge,. Other amino acid residues of the annexin component, such as cysteine, may also be employed for binding purposes. Alternatively a maleimide-S-(CH₂)ₙCO- binding facilitation moiety-extender combination may be employed to link the sugar residue with the lysine. A example of a conjugate joined by a trifunctional linker is discussed in Example VII.

When the chelate component of the conjugate is characterized by greater than one functional group available and suitable for conjugation, the galactose cluster may be linked to the chelate component which, in turn, is linked to the annexin component of the conjugate via two or more bifunctional linkers. Preferably, the annexin component of the conjugate is attached last in the formation of a galactose cluster-chelate-annexin conjugate. Suitable bifunctional linkers and linking methodologies can be identified and employed by one skilled in the art. An example of such a chelate and such a conjugation methodology is set forth in Example V.

Annexin conjugation to the hexose cluster and the chelate via chemical methods can occur either prior to (post-formed approach) or following (pre-formed approach) complexation of a radiometal with the chelate. The chemical conjugation is preferably conducted following radiometal complexation, unless the chelate employed in the conjugate is capable of binding the radionuclide with rapid kinetics at room, temperature.

Annexin V is radiolabeled with an imaging radionuclide for use in the present invention. Radionuclides useful within the present invention include gamma-emitters, positron-emitters, Auger electron-emitters, X-ray emitters and fluorescence-emitters. Radionuclides suitable for use in the present invention are known in the art and include ⁶⁴Cu, ¹⁸⁶Re, ¹⁸⁸Re, ¹⁰⁰Pd, ²¹²Bi, ²¹²Pb, ¹⁰⁹Pd, ⁶⁷Cu, ^{99m}Tc, ⁹⁴Tc, ⁹⁵Ru, ¹⁰⁵Ru, ⁹⁹Rh, ¹⁰⁵Rh, ¹¹¹In, ¹⁵³Sm, ¹⁷⁷Lu, ¹⁷⁰Lu, ¹⁸⁹Pt, ¹⁹³Pt, ¹⁹⁹Au, ¹⁹⁷Hg.

Tc-99m is a preferred radionuclide for the practice of the present invention. Tc-99m has been stably bound to annexin V in accordance with the present invention at both low and high specific activity (0.53 µCi/µg *-* 101.2 µCi/µg). Adequate radiochemical yields and good radiochemical purities were obtained. Activated platelet binding studies were also conducted, and the radiolabeled annexin V conjugates bound to activated platelets well.

N₂S₂ and N₃S chelates are known in the art. For example, preferred N₂S₂ chelates are described in U.S. Patent No. 4,897,225, and preferred N₃S chelates are described in U.S. Patent No. 4,965,392. The present inventors have applied this stable chelation technology to exploit the thrombus-targeting ability of annexin molecules, thereby providing an imaging agent which is able to rapidly visualize vascular thrombi in vivo. The radiolabeled annexins, radiolabeled annexin-galactose conjugates and radiolabeled annexin-galactose cluster conjugates of the present invention can be used to obtain thrombus images which reduce or eliminate the high level of background radioactivity that results from metabolically degraded radiolabeled conjugate. The radiolabeled annexins, radiolabeled annexin-galactose conjugates and radiolabeled annexin-galactose cluster conjugates also avoid clinically unacceptable toxicity to non-target cell sites. Tc-99m radiolabeled annexin V performed better than I-123 in the pig studies set forth in Example III hereof.

Radiolabeling of annexin V with a radionuclide using an N₂S₂ or N₃S chelate may be conducted using either a pre-formed or a post-formed approach. That is, the radionuclide is either complexed within the chelate prior to (pre-formed) or following (post-formed) conjugation of the chelate to annexin V. The pre-formed approach is preferred and suitable procedures therefor are set forth in Examples I, II, and IV.

For embodiments of the present invention wherein chelates exhibiting rapid complexation of radionuclide at room temperature, chelates of the following structure may be employed: 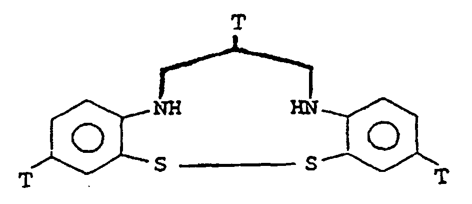 wherein one or more T substituents incorporate a functional group available and suitable for conjugation with another conjugate component. In the example noted above, a functional group, such as an amine, is capable of reacting with the lysine carboxyl moiety or an activated ester derivative thereof. Alternatively, an active ester bearing chelate may be conjugated to an amino functional group, for example. One skilled in the art is versed in the nature and specificity of functional moieties, and such nature and specificity are also discussed herein.

Annexin molecules may be modified by the addition of from 2 to 6 terminal amino acid residues to facilitate the conjugation reaction between the annexin molecule and the chelate or between the annexin molecule and a galactose residue or between the annexin molecule and either a linker or a hexose cluster. For example, terminal amino acid residues may be added to provide a sulfhydryl group or to provide a group capable of derivatization to a maleimide group, with such sulfhydryl and maleimide groups available for the conjugation reaction. This modification may be made by protein chemistry methods or via production of an appropriate fusion protein or other techniques useful therefor.

Radiolabeled annexin-galactose cluster conjugates of the present invention offer an additional advantage over the previously prepared I-123-labeled annexins, in that they are amenable to packaging in a cold kit. That is, the annexin or annexin-galactose or annexin-galactose cluster or galactose cluster and chelate components may be individually vialed and provided separately from the Tc-99m component (and, possibly, vialed separately from each other). When a patient requiring a thrombus image is identified, a cold kit may be ordered or retrieved from storage; Tc-99m may be obtained from a radiopharmacy or other source thereof; the pre-formed or post-formed chelation/complexation process is performed; the radiolabeled annexin-galactose cluster conjugate is administered to the patient; and the patient is subsequently imaged. For radiolabeled annexin-galactose cluster conjugates, a chelate-annexin-galactose cluster conjugate is preferably prepared and vialed in the kit, although annexin-galactose cluster/chelate two component or other multi-component kits may also be used.

Lyophilization and vialing in a sterile, pyrogen-free environment of the conjugate components may be accomplished via techniques, known to persons skilled in the art of good manufacturing practices, particularly as such practices relate to biological materials.

Radiolabeled annexin-galactose cluster conjugates of the present invention are administered in such amounts as to deliver a diagnostically effective amount of radionuclide to target sites. Appropriate administered doses depend on a variety of factors that are largely patient specific, and practitioners in the art will consider such factors in the practice of the present invention. The components of the radiolabeled annexin-galactose cluster conjugates also impact dose amounts in ways that are known to or routinely ascertainable by practitioners in the art. In general, radiolabeled annexin-galactose cluster conjugate is administered to large mammals at a dose ranging between 0.3 and 300 micrograms/kg body weight of the recipient, with from 3 to 10 micrograms/kg preferred, depending upon the physiological characteristics of the patient and the ailment involved or suspected. A practitioner in the art is capable of identifying an appropriate dose and administration route for a given recipient with a given ailment. ,

Radiolabeled annexin-galactose cluster conjugates of the present invention may be administered in any convenient manner therefor. For example, intravenous infusion may be employed to administer radiolabeled annexin-galactose cluster conjugates. Other routes of administration also find utility in the practice of the present invention. Exemplary additional administration routes are injection by the arterial (e.g., coronary artery), intracoronary, intralymphatic, intrathecal, or other intracavity routes.

After administration of the radionuclide, depending upon the nature of the radionuclide and the purpose of the administration, the recipient may be subject to various procedures for detection of radioactive emissions from the site or sites at which the radionuclide localizes. For example, conjugates containing Tc-99m are imageable by a gamma camera.

The invention is further described through presentation of the following examples.

### Example I (Referential)

### Procedure for Radiolabeling an Annexin - N₂S₂ Chelate Conjugate

Annexin V can be isolated from a variety of tissue extracts, such as liver, lung and placenta, in accordance with procedures set forth in Funakoshi et al., Biochem. 26: 8087-92, 1987); Tait et al., Biochem. 27: 6268-76, 1988; and U.S. Patent No. 4,937,324, for example. In addition, annexin V can be expressed in E. coli, as described by Tait et.al., Archives of Biochemistry and Biophysics 288: 141-44, 1991.

Annexin V was radiolabeled with Tc-99m by using a diamide dimercaptide N₂S₂ chelate in accordance with the OncoTrac® Small Cell Lung Cancer Imaging Kit labeling procedure described in J. Nucl. Med. 32: 1445-51, 1991.

A preferred method for radiolabeling annexin V with Tc-99m constitutes a modified OncoTrac® kit procedure using C-18 Baker purified Tc-99m-N₂S₂-TFP. In this procedure, an acidified active ester solution was prepared by adding 0.16 ml of 0.2M hydrochloric acid: glacial acetic acid (14:2 ratio) to 0.6 ml of 2,3,5,6,-tetrafluorophenyl 4,5-bis-(S-1-ethoxy-ethylmercaptoacetamido)pentanoate (0.3 mg, 0.0005 mole freshly dissolved in 0.9 ml of isopropyl alcohol). Then 0.5 ml of this solution was added to 1.1 ml of Tc-99m-gluconate (prepared from 0.12 mg SnCl₂ 2 H₂O, 5.0 mg sodium gluconate at pH 6.1-6.3, and 100 mCi/ml of [Tc-99m] pertechnetate, i.e., the first step in the OncoTrac® kit labeling procedure). The reaction mixture was heated at 75 C for 15 minutes followed by cooling on ice. The resulting Tc-99m transchelated tetrafluorophenyl active ester derivative of Tc-99m-4,5-bis(thioacetamido)pentanoate was, optionally and preferably diluted with 2 ml water and purified by loading the reaction mixture on a conditioned C-18 cartridge (J.T. Baker), washing with 2.0 ml water eight times followed by drying the column for 5 minutes, and eluting with 100% acetonitrile. The solvent was evaporated under a steady stream of N₂. Then 0.15 ml of phosphate buffered saline (PBS), 0.15 ml of annexin V at 2.35 mg/ml, and 0.2 ml of 0.2 M , bicarbonate (pH 10.0) were added for conjugation to Tc-99m-N₂S₂. After 20 minutes at room temperature, the Tc-99m-N₂S₂-annexin V conjugate was purified by passage through a G-25 SEPHADEX® (PD-10) column (available from Pharmacia) equilibrated with PBS. Fractions (1.0 ml) were collected, and those fractions containing annexin V were pooled. Protein concentration was determined by UV absorption at 280 nm. Tc-99m-annexin V (300 -350 mg) conjugate solution was diluted and stored in PBS containing bovine serum albumin (BSA) at a final concentration of 15-20 mg BSA/ml PBS prior to injection.

### Example II (Referential)

### Procedure for Radiolabeling an Annexin - N₃S Chelate Conjugate

S-benzoylmercaptoacetylglycylglycylglycine (S-benzoyl MAG₃) is prepared in accordance with the procedures described in U.S. Patent No. 4,965,392. Then 25 micrograms of S-benzoylmercaptoacetylglycylglycylglycine is dissolved in 0.10 ml of 1.0 M carbonate buffer (pH 12). Then 75 mCi of Tc-99m pertechnetate is added in about 1.0 ml followed by 1.0 mg of freshly dissolved sodium dithionite (10 mg/ml). This mixture is heated at 100 C, plus or minus 4 C, for 3 minutes, then is cooled in an ice bath for 5 minutes to give Tc-99m-MAG₃ as determined by ITLC (CH₃CN solvent); anion exchange HPLC (Beckman AX, 10 micron 0.01M Na₂SO₄/0.01M Na₃PO₄, pH 7.0); and reverse phase HPLC (Beckman ODS, 5 micron 2% CH₃CN/0.01M Na₃PO₄, pH 7.0).

The Tc-99m-MAG₃ complex in carboxylate form is then esterified; 0.20 ml 1N HCl, 0.30 ml of 0.2M phosphate buffer pH 6.0, 10.0 mg 2,3,5,6-tetrafluorophenol (TFP) in 0.01 ml 90% CH₃CN, and 12.5 mg of EDC (1-(3-dimethylaminopropyl)-3-ethylcarbodiimide hydrochloride) in 0.10 ml of 90% CH₃CN are combined, and the reactants are mixed at room temperature (25 C, plus or minus 2 C) for 1 hour. At this point, a yield of Tc-99m-MAG₃-TFP ester as determined by ITLC (CH₃CN solvent); anion exchange HPLC (Beckman AX, 10 micron 0.01M Na₂SO₄/0.01M Na₃PO₄, pH 7.0); and reverse phase HPLC (Beckman ODS, 5 micron 34% CH₃CN/0.01M Na₃PO₄, pH 7.0). The preparation is purified using a C-18 Baker column. The reaction mixture is, optionally and preferably diluted with 2 ml water and loaded in the column, washed two times with water and then eight times with 10% C₂H₅OH/0.01M Na₃PO₄, pH 7.0. The product is eluted with CH₃CN, and the solvent is removed prior to conjugation with annexin V.

The conjugation of the active ester to annexin V is carried out by adding annexin V in a phosphate buffer, pH 9.5, to the Tc-99m-MAG₃-TFP ester. The reaction is carried out for at least 30 minutes, and the desired radiolabeled annexin product is obtained by passage through a PD-10 gel filtration column.

### Example III (Referential)

### Thrombus Imaging With a Radiolabeled Annexin

### A. Animal Preparation - LA Vascular Thrombi.

Fasting 25-30 kg Yorkshire swine were sedated with intramuscular Telazol (5-10 mg/kg) (commercially available from AVECO Co.) and commercially available Atropine (1 mg, Elkins-Sinn, Inc., Cherry Hill, NJ). Surital (200 mg) (commercially available from Abbott Laboratories) anaesthesia was administered intravenously. The animals were intubated and given inhalation anaesthesia of 1.5-2% halothane (commercially available from Abbott Laboratories) and O₂ sufficient to obtain a deep level of anesthesia and physiologic arterial blood gases. Continuous electrocardiographic monitoring was instituted using alligator clip electrodes. A cut down in the neck region was conducted, and an 8 french catheter (USCI Co, Billerica, MA) was placed in the right common carotid artery for blood pressure and arterial blood gas monitoring as well as for blood sampling.

The swine were placed in a right lateral decubitus position, and a lateral thoracotomy was performed to expose the heart. The incision was held open by a thoracotomy retractor. The pericardium was opened, and the left atrial appendage was isolated from the, left atrium by a vascular cross- clamp. Rubber tipped forceps were used to gently crush the appendage. Five minutes later, ricinoleate (1 mg, ICN Pharmaceuticals, Costa Mesa, CA) and thrombin (50 mg, Johnson and Johnson Co., Arlington, Texas) were injected into the LAA (left atrial appendage) using a 27 Ga needle. The, cross-clamp was removed 10 minutes later.

One hour after the crush injury, Tc-99m-annexin.V, prepared in accordance with Example I above, was administered as an intravenous bolus dose in an ear vein. The intravenous line was then flushed with saline. In 7 animals, I-125 labeled ovalbumin was administered as a non-specific control preparable, for example, by the procedure described by Fraker et al., Biochem. Biophys. Res. Commun. 80: 849-57, 1978). Briefly, I-125-radiolabeled ovalbumin was prepared by the Iodogen method, employing 600 g ovalbumin (Sigma Chemical Co., St. Louis, Missouri) and NaI-125 (2mCi, 0.92 nmol).

Image acquisition was performed as described below. At the end of the experimental procedure (generally about 150 minutes), the animals were sacrificed by an intravenous bolus dose of 80 mEq of KCl while the animals were still under general anaesthetic. A final blood sample was taken for well counting. The heart was rapidly excised, washed free of blood and dissected into samples for well counting. Additional samples of carotid artery, lung, liver, spleen, muscle and kidney were obtained in some animals.

B. Controls. Five different types of controls were used: open chest sham; closed chest sham; ovalbumin; indium platelets; and non-specific Tc-99m- , labeled antibody.
1. Open Chest Sham. In three animals, the heart was exposed as above, but the left atrium was not isolated, crushed or injected with ricinoleate/thrombin. Marker images with a cobalt marker were performed as described below, and the Tc-99m-annexin V was injected 30-60 minutes after LAA exposure. Imaging and sample acquisition were identical to that described in A above.
2. Closed Chest Sham. In seven animals, an ear intravenous line was established. No thoracotomy was performed. Sedation and anesthesia were identical to that described in A above. The Tc-99m-annexin V (+/other control radionuclides, such as I-125 ovalbumin or In-111-platelets) was administered and image acquisition was performed.
3. Ovalbumin. 1-125 ovalbumin was administered in 7 animals as a negative control protein. ovalbumin has a molecular size similar to that of annexin, and exhibits a slightly slower blood clearance.
4. Indium Platelets. In-111 platelet labeling was performed in 7 animals as a positive well counting label. In-111 radiolabeled platelets were prepared in accordance with the procedure described by Stratton et al., Am. J. Cardiol. 47: 874, 1981 and Stratton et al., Circulation 69: 561, 1984. Imaging was not attempted because of the long serum half-life of the In-111-platelets.
5. Non-specific Tc-Labeled Antibody. In a single experiment, a left atrial (LA) thrombi was created by the above method, but Tc-99m-Annexin V was not administered. Instead, a Tc-99m-Fab fragment of an antibody designated as NR-LU-10 was administered. NR-LU-10 is a 150 kilodalton molecular weight IgG2b monoclonal antibody that recognizes an approximately 40 kilodalton glycoprotein antigen expressed on most carcinomas. NR-LU-10 is a well characterized pancarcinoma antibody that has been safely administered to over 565 patients in human clinical trials. NR-LU-10-Fab is prepared in accordance with known techniques and is radiolabeled in accordance with the procedures described in J. Nucl. Med. 32: 1445-51, 1991 and by the modified C-18 Baker purified Tc-99m-N₂S₂-TFP procedure described in Example I for preparing radiolabeled annexin V. This Tc-99m-Fab conjugate was designed as a negative control for both well counting and imaging.

C. Imaging. A cobalt marker was placed on the exposed surface of the LAA and held in place with surgical tape affixed to the thoracotomy retractor. The tape was adjusted so that the marker generally moved with the LAA with each cardiac cycle. Marker images were acquired for 10 seconds in each planar view and 10 seconds in each tomographic slice. The cobalt marker was then removed.

A General Electric Starport camera with a general purpose collimator was used to acquire the Tc-99m images. Five minute planar acquisitions were performed sequentially in the left lateral, 45 LAO, and anterior views. These were followed by a 10 minute tomographic acquisition. This full set of 3 planar and 1 tomographic acquisitions was repeated for a total of 5 sets. Care was taken not to move the pig or imaging gantry during the entire imaging sequence.

Images were recorded on a Microdelta computer slaved to a VAX mainframe system. Images were stored on tape or on the VAX hard drive. Planar image analysis consisted of first viewing the image with the marker and recording the marker position on the viewing terminal screen. The first image acquired after Tc-99m-annexin V injection was used to define the cardiac blood pool. Each subsequent image was viewed and analyzed using the marker and initial blood pool as references. Each image was scored as positive, equivocal or negative.

Thirteen animals had left atrial thrombi created and were imaged as described above. Twelve animals had Tc-99m-annexin V injected for imaging, and one animal had a non-specific Tc-99m Fab injected as a control. Closed chest imaging was performed in 7 animals without atrial injury, and open chest sham experiments were performed in 3 animals. In animals with atrial thrombi, all planar images taken at less than 35 minutes after administration of Tc-99m-annexin V were negative. Nine of the atrial thrombi planar images taken at greater than 70 minutes were positive, 1 was equivocal and 2 were negative. All of the tomographic images of atrial thrombi were either positive (n=10) or equivocal (n=2) at imaging times of greater than 2 hours post injection. The average time in which the planar images became positive following administration of Tc-99m-annexin V was 82 minutes (35-135 minutes).

None of the closed chest control animals had positive images. One of the three open chest shams had a positive image at 85 minutes. This false positive is believed to result from the production of surgically-induced thrombi.

These results indicate that intravenous administration of Tc-99m-annexin V permitted acquisition of diagnostic images identifying atrial vascular thrombi within a short time period following conjugate administration.

D. Sample Collection. Samples (both blood and tissue), as described above, were weighed and placed in vials for immediate counting of Tc-99m. After the Tc-99m had decayed (typically at 5-7 days), the samples were re-assayed to obtain the I-125 counts. Each vial was counted for 1 minute. The counts were corrected for decay, then for weight, and recorded as counts per minute per gram. The counts per minute per gram for each sample were then normalized by dividing the counts per minute per gram of the final blood specimen. Consequently, the results for each sample were calculated in a ratio with the last blood sample, permitting meaningful comparison between animals. This procedure was performed for all radionuclides in a given experiment.

Well counting ratios were obtained for a variety of tissue samples. For the injured tissues and thrombi, multiple specimens were usually taken from the same animal. In those cases, the maximum ratio for any one specimen is reported, as well as the average of all specimens taken. The maximum for each animal was averaged across all animals and is reported as the Maximum Anx-V Ratio.

These results indicate that Tc-99m-annexin V localizes preferentially to atrial thrombi and injured left atrium, with the highest non-target localization occurring in the kidney. The kidney level is at least partially indicative of excretion of Tc-99m-annexin V via the renal route.

### Example IV (Referential)

### Radiolabeled Annexin-Galactose Conjugates

A. Annexin V -Preparation from human placenta and by expression in *E. coli*. Annexin V was purified from human placenta to a final purity of 99% as described by Funakoshi et. al., Biochemistry 26, 5572-78, 1987 and Tait et. al., Biochemistry 27, 6268-76, 1988.

Alternatively, annexin V was obtained, but not employed in the following experiments, by expression in *E*. *coli*. This expression was conducted as follows.
1. Preparation of bulk fermentation supernatant. Standard molecular biology techniques were used to express annexin V in *E*. *coli*. More specifically, the protein coding region of annexin V cDNA (see, Funakoshi et. al. referenced above) was inserted into the NdeI/BamHI site of plasmid pET-12a (Novagen, Madison WI). The selected plasmid was used to transform *E. coli* strain BL21 (DE3) (Novagen).
   Individual colonies (clones) were isolated on Luria Broth plates containing 50 g/ml ampicillin or carbenicillin, as discussed in Sambrook et al., "Molecular Cloning Laboratory Manual," 2nd ed., Cold Springs Harbor Laboratory Press, 1989. For production, cultures of the selected clones containing the desired plasmid were grown overnight at 37 C with shaking in Terrific Broth (see, Tartof et. al., Bethesda Res. Lab. Focus, 9: 12, 1987) containing 50 µg/mL carbenicillin (commercially available from Sigma Chemical Co., St. Louis, Missouri). The culture was diluted 1:20 in the same medium and incubated at 37 °C with shaking for 18-24 hr. The bacteria were harvested by centrifugation, washed once with an equal volume of 0.05 M Tris-HCl, 0.15 M sodium chloride, pH 8, and stored as pellets at -20 C. Pellets were thawed and resuspended with 10-15% (w/v) cold 0.05 M Tris-HCl, 0.02M Na₄EDTA, pH 7.2. The suspension was sonicated for 4 min on ice, centrifuged, and the supernatant stored at -70 C.
2. Purification of annexin V from bulk fermentation supernatant. After thawing, the supernatant was filtered using a 0.1 µm hollow fiber unit (commercially available from A/G Technology, Needham, MA), and the conductivity adjusted with concentrated sodium chloride to equal 0.02 M Tris-HCl, 0.5 M sodium chloride, pH 8. Polyethyleneimine-WP (J.T. Baker, Phillipsberg, NJ) was suspended in 0.02 M Tris-HCl, 0.5 M sodium chloride, pH 8, and 1 g of resin per 10 ml of bulk fermentation supernatant was added to the filtered supernatant. After stirring for 30 min, the supernatant was removed, exchanged into 0.02 M Tris-HCl, pH 8, and applied to a Q-SEPHAROSE® HP column (Pharmacia, Piscataway, NJ) equilibrated in the same buffer. The column was developed with a step gradient of 0-0.5 M sodium chloride. Fractions were assayed for annexin V by size exclusion HPLC, and fractions containing A₂₈₀ absorbing material of appropriate size were pooled. The semi-purified annexin V was exchanged into PBS and concentrated by ultra-filtration. Ge1 filtration over SEPHACRYL® 100 HP (Pharmacia) equilibrated in PBS yielded the purified protein. The protein concentration was adjusted to 1 mg/ml, sterile 0.2 µm filtered, and stored at 2-8 °C.

### B. Derivatization of Annexin V with Galactose.

The general method of Lee et al., Biochemistry 15: 6268-76, 1976 was followed.
a. Preparation of 2-imino-2-methoxyethyl-1-thio--D-galactopyranoside.
A 1.0 M solution of cyanomethyl-2,3,4,6-tetra-O-acetyl-1-thio- -D-galactopyranoside (commercially available from Sigma Chemical Company, St. Louis, MO) was prepared by dissolving 1.83 g (4.5 mmole) in 4.5 ml anhydrous methanol with heating to 50 °C. The solution was maintained at 50 °C, and 0.1 ml (0.45 mmole) of 25% sodium methoxide in methanol (commercially available from Aldrich Chemical Company, Milwaukee, WI) was added with continuous stirring. After 6 hr at 50 °C, the methanol was removed under reduced pressure yielding the galactose methyl imidate as a colorless, viscous oil.
b. Galactosylation of annexin V
The galactose methyl imidate was offered to annexin V in molar ratios of 300:1, 150:1, and 75:1. For the 300:1 offering, 3.35 mg of galactose methyl imidate in methanol was taken to an oil under a stream of nitrogen. To the oil was added 2.53 mg of annexin V in 0.05 M HEPES buffer (commercially available from Sigma Chemical Co., St. Louis, Missouri), pH 7.4 and 0.04 ml of 0.5 M sodium borate buffer, pH 8.5. The solution was mixed for 1 hr at room temperature by tumbling the reaction vessel, and was then allowed to stand at room temperature overnight (approximately 18 hr). The reaction mixture was diluted with 0.4 ml PBS, transferred to dialysis tubing (6000-8000 MW cutoff), and dialyzed 24 hr against PBS. After removing the material from the dialysis bag, the solution was filtered through a 0.2 µm syringe filter. The other offering levels were conducted analogously.
C. Characterization of Galactose-Derivatized Annexin V. Protein concentration was determined using A₂₈₀ of 0.6 for a 1 mg/ml solution of annexin V. The number of galactose residues per molecule of annexin V was determined by measuring the total number of reactive amines on annexin V before and after reaction with galactose methyl imidate using trinitrobenzenesulfonic acid, as described by Habeeb, Analytical Biochemistrv 14: 328-36, 1966.

| Offering Ratio | Substitution Ratio |
|---|---|
| 300:1 | 4.7:1 |
| 150:1 | 2.3:1 |
| 75:1 | 1.1:1 |

The ability of galactose-modified annexin V to bind to activated platelets was assessed by determining its ability to inhibit the binding of unmodified, ¹²⁵I radiolabeled annexin V to freshly isolated human platelets, following the method of Thiagarajan and Tait J. Biol. Chem. 265: 17240-43, 1990. The following table shows the results of the competition assay, both in absolute value (left column) and in a value normalized to 100% for unmodified annexin V (right column).

| Substitution Ratio | Competition (% of control) | Competition (% of control) |
|---|---|---|
| 4.7 | 0 | 0 |
| 2.3 | 5.7 | 7.6 |
| 1.1 | 21.4 | 28.7 |
| unmodified | 74.5 | 100 |

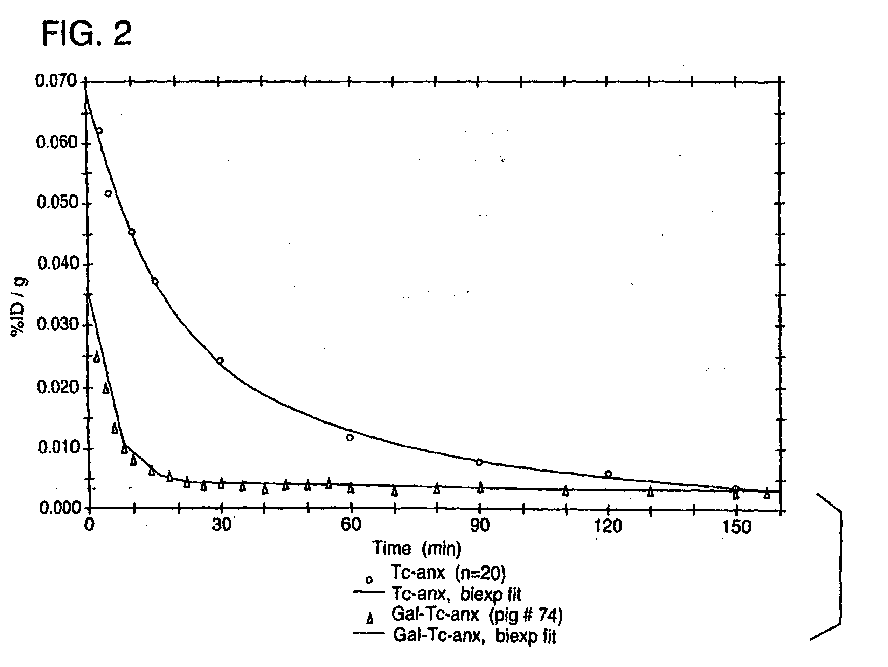

D. Preparation of Tc-99m-Annexin V-Galactose. Annexin V-galactose was radiolabeled with technetium-99m using a diamide dimercaptide (N₂S₂) chelate as described by Kasina et. al., J. Nucl. Med. 32: 1445-51, 1991. The preformed active ester chelate was diluted with 2.0 ml water and purified before conjugation to annexin V-galactose using a modified conditioned C-18 column (commercially available from J.T. Baker), as described by Fritzberg et. al., Proc. Natl. Acad. Sci. USA 85: 4025-29, 1988, washing with 2.0 ml water eight times followed by drying the column for 5 min and eluting with 100% acetonitrile. The solvent was removed under a steady stream of N₂. Then 0.15 ml of PBS, 0.35 ml of annexin V-galactose (4.7:1 galactose:annexin V) at 1.0 mg/ml, and 0.2 ml of 0.2M bicarbonate buffer (pH 10.0) were added for conjugation to Tc-99m-N₂S₂-TFP ester. After 20 min at room temperature, the Tc-99m-annexin V-galactose conjugate was purified by passage through a G-25 SEPHADEX® PD-10 column (commercially available from Pharmacia) equilibrated with PBS.
Fractions of 1.0 ml were collected, and fractions containing annexin V were pooled. Protein concentration was determined by UV absorption at 280 nm. The radiochemical yield of the conjugate was 35.3%. The radiochemical purity was 99.8%, as assessed by instant thin layer chromatography on silica gel impregnated glass fiber sheets developed in 12% w/v aqueous trichloroacetic acid. The ITLC sheets were cut into two halves, and each half was counted in a gamma counter or a dose calibrator. The radiolabeled annexin V-galactose conjugate precipitated at the origin, and any non-protein-bound radioactivity migrated with the solvent front in this solvent system. Tc-99m-annexin V-galactose conjugate (300-350 g) solution was diluted and stored in PBS containing bovine serum albumin (commercially available from Sigma Chemical Co.) at a final concentration of 15-20 mg/ml PBS prior to intravenous injection.
E. Blood Clearance of Tc-99m-Annexin V and of Tc-99m-Annexin V-Galactose. An animal model, as previously described in Example III, was employed to evaluate blood clearance of the molecules. For N=20 swine, the blood clearance (Figure 2) is biphasic (biexponential) :

| | | | |
|---|---|---|---|
| %ID/g | a = 0.0389 | t1/2 | a = 9.6 min |
| %ID/g | b = 0.0300 | t1/2 | b = 46 min |

a + b = 0.0689, therefore 57% (0.0389/0.0689) of the injected dose/gram of blood is cleared in the faster a phase and 43% of the ID/g is cleared in the slower b phase.

Blood clearance of Tc-99m-annexin V-galactose in the swine (Figure 2) is also biphasic (biexponential) :

| | | | |
|---|---|---|---|
| %ID/g | a = 0.0313 | t1/2 | a = 3.5 min |
| %ID/g | b = 0.0045 | t1/2 | b = 160 min |

a+b = 0.0358, therefore 87% (0.0313/0.0358) of the injected dose/gram of blood is cleared very quickly in the early a phase. This more rapid clearance reduces the background radioactivity in the blood compartment, i.e., the noise, and therefore improves the signal to noise ratio. Consequently, thrombus imaging can be achieved at shorter time points.

### EXAMPLE V

### Chelate Preparation

4,4 - Diethoxycarbonylpropyl - 1,3 - dianiline: A stirred solution of 2.065 g (1.25 mole) ethyl-4-amino benzoate, 14.35 mL (0.125 mole) 1,3-di-iodopropane and 10.5 g (0.125 mole) sodium bicarbonate in 500 mL dry DMSO is heated at 100°C for 3 hours under nitrogen. Upon cooling, the mixture is poured into 2 L of ice water with stirring, and the resulting precipitate is collected by filtration. The precipitate is then washed with glacial acetic acid (14 x 75 mL) until all of the starting ethyl-4-aminobenzoate has been removed. After drying *in vacuo*, the product, thus obtained, is used in the next step without further purification.

1,3-Di(2-imino-6-ethoxycarbonylbenzthiazolyl-3-)propane: Ammonium thiocyanate (16.5 g, 0.217 mole) is added to a magnetically stirred suspension of 4,4-diethoxycarbonylpropyl-1,3-dianaline (10.0 g, 0.27 mole) in 1500 mL glacial acetic acid. A solution of bromine (34.6 g, 0.216 mole) in 100 mL glacial acetic acid is then added dropwise to the suspension with stirring at room temperature. After stirring the reaction mixture overnight at room temperature, the dihydrobromide salt of the crude product is collected by filtration and dried. The product is isolated by dissolving the crude product in hot water, adjusting to basic pH with the addition of saturated sodium bicarbonate solution, collecting the precipitate by filtration, and drying *in vacuo*.

N,N'-Bis(2-disulfidyl-4-hydroxycarbonylphenyl)-1,3-propyldiamine: Solid potassium hydroxide (20.0 g, 0.357 mole) is added to a suspension of 1,3-di(2-imino-6-ethoxycarbonylbenzthiazolyl-3-)propane (1.0 g, 0.002 mole) in 40 mL distilled water, and the resulting mixture is heated at 120°C for 12 hours. Complete dissolution occurs after 1 hour. The reaction mixture is then cooled in an ice bath, and the pH is adjusted to 5.0 with 5.0 N acetic acid. The aqueous solution is then extracted with three 100 mL portions of ethyl acetate. The combined ethyl acetate extracts are dried over anhydrous sodium sulfate, and the drying agent is filtered. Removal of solvent yields the product.

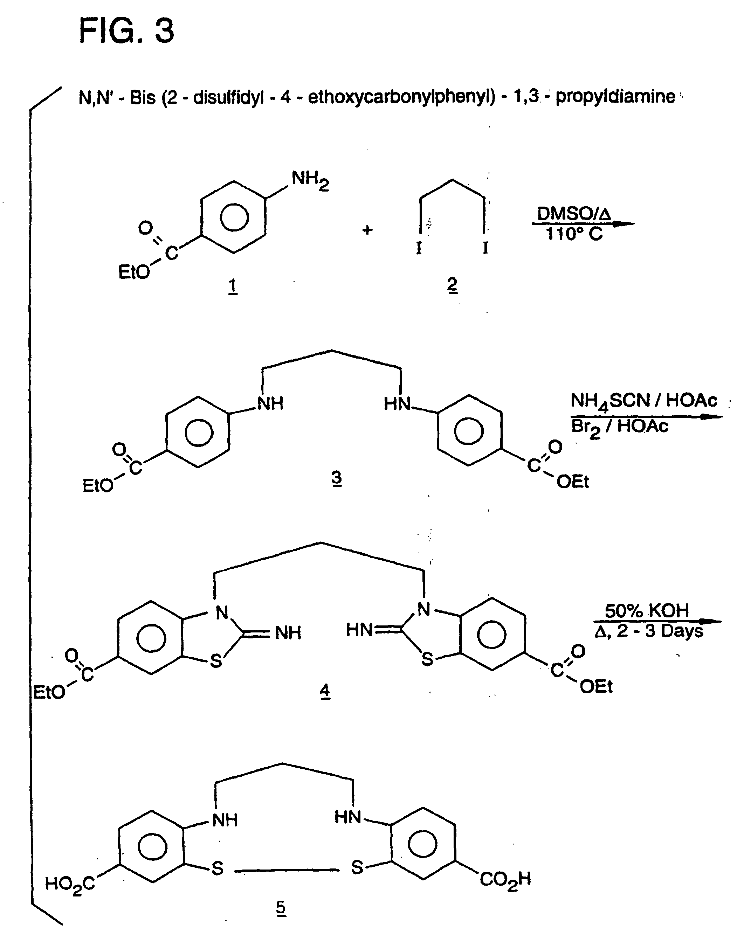

To this point, this synthesis is shown schematically in Figure 3.

N,N'-bis(2-disulfidyl-4-hydroxycarbonylphenyl)-1,3-propyldiamine mono-(methyl-aminocaproate) adduct. To a mixture of N,N'-bis(2-disulfidyl-4-hydroxycarbonylphenyl)-1,3-propyldiamine and 2-3 equivalents of methyl 6-aminohexanoate hydrochloride (prepared as described below in Example VI) in dimethylformamide is added 10 equivalents of triethylamine followed by 0.9-1.0 equivalent of BOP (benzotriazol-1-yloxy-tris(dimethyl-amino)-phosphonium hexafluorophosphate). The mixture is stirred at 15-30°C for 2-24 hours and then concentrated. The residue is diluted with deionized water, and the pH is adjusted to approximately 2 with 1 N aqueous hydrochloric acid. The mixture is again concentrated. The residue is chromatographed on silica gel. The chromatographic fractions containing product are combined and concentrated to afford the product.

This chelate is amenable to use with a suitable trifunctional linker or a pair of bifunctional linkers to form a radiolabeled annexin-galactose cluster conjugate of the present invention. The use thereof with a pair of bifunctional linkers is addressed in the following example.

### EXAMPLE VI

### Radiolabeled Annexin-Galactose Cluster Conjugates Bifunctional Linker Approach

### A. Preparation of an Eight Galactose Cluster.

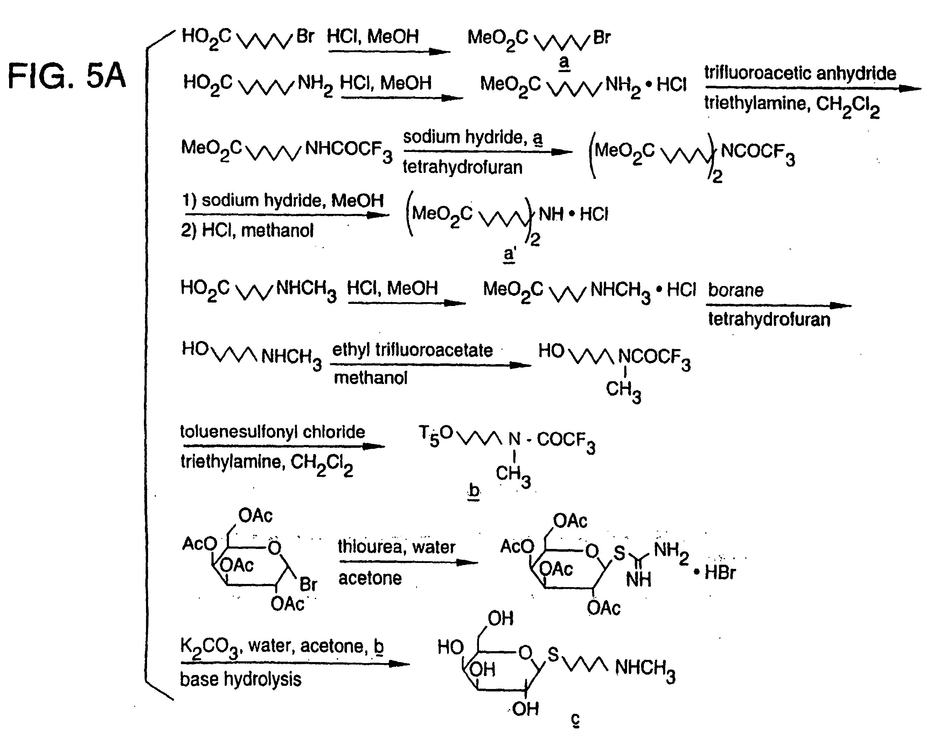

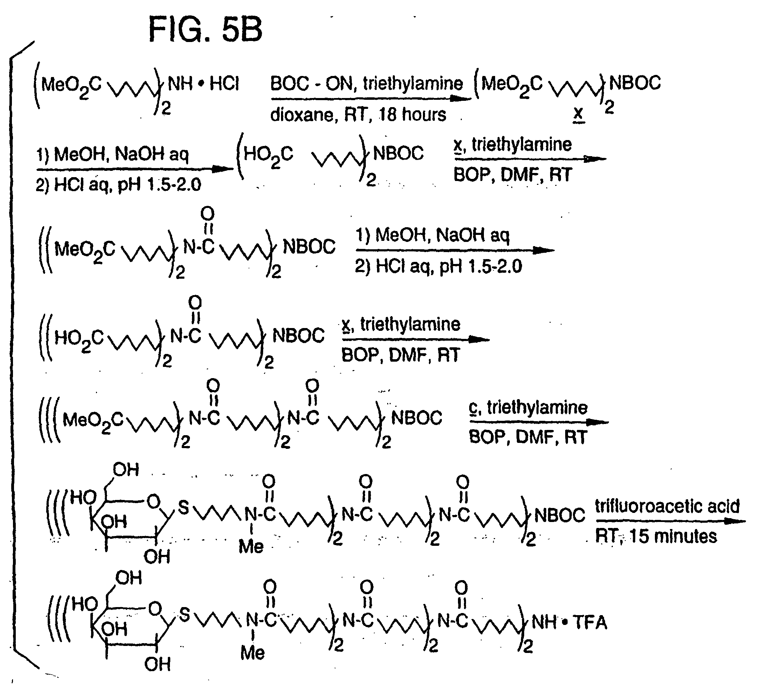

This procedure is schematically shown in Fig. 5.

Methyl 6-bromohexanoate. To a 1 L round bottom flask, charged with 20 g (102.5 mmol) of 6-bromohexanoic acid and 500 mL of methanol, was bubbled hydrogen chloride gas for 2-3 minutes. The mixture was stirred at room temperature for 4 hours and concentrated to afford 21.0 g of the product as a yellow oil (99%): ¹H-NMR (200MHz, d₆-DMSO); 3.57 (s, 3H), 3.51 (t, 2H), 2.30 (t, 2H), 1.78 (pentet, 2H), and 1.62-1.27 (m, 4H) ppm.

Methyl 6-aminohexanoate hydrochloride. To a 1 L round bottom flask, charged with 40.0 g aminocaproic acid, was added 500 mL of methanol. Hydrogen chloride gas was bubbled through the mixture for 5 minutes, and the mixture was stirred at room temperature for 5 hours. The mixture was then concentrated via rotary evaporation and then under full vacuum pump pressure (<0.1 mm Hg) to afford 55 g of the product as a white solid (99%): ¹H-NMR (200 MHz, CD₃OD); 3.67 (s, 3H), 3.02 (t, 2H), 2.68 (s, 3H), 2.48 (t, 2H), and 2.03-1.87 (pentet, 2H) ppm.

Methyl 6-(trifluoroacetamido)-hexanoate: To a 1 L round bottom flask, charged with 25.0 g (138 mmol) of methyl 6-aminohexanoate hydrochloride and 500 mL of methylene chloride, was added 24 mL (170 mmol) trifluoroacetic anhydride. The mixture was cooled in an ice bath, and 42 mL (301 mmol) of triethylamine was added over a 25-30 minute period. The mixture was stirred at 0°C to room temperature for 2 hours and then concentrated. The residue was diluted with 150 mL of diethyl ether and 150 mL of petroleum ether, and the resulting solution was washed first with 1 N aqueous HCl (3 x 150 mL) and then with saturated aqueous sodium bicarbonate (3 x 150 mL). The organic phase was dried over magnesium sulfate, filtered and concentrated to give 32.9 g of the product as a pale yellow oil (99%): ¹H-NMR (200MHz, d₆-DMSO); 9.39 (m, 1H), 3.57 (s, 3H), 3.14 (q, 2H), 2.29 (t, 2H), 1.60-1.38 (m, 4H), and 1.32-1.19 (m, 2H) ppm.

N,N'-Bis(6-methoxycarbonylhexyl)amine hydrochloride. To a 500 mL dry round bottom flask, charged with 12.0 g (50.0 mmol) of the secondary amide, methyl 6-(trifluoroacetamido)-hexanoate, and 250 mL of dry tetrahydrofuran, was added 2.2 g (55 mmol, 1.1 equiv) of 60% sodium hydride. The mixture was stirred at room temperature for 30 minutes and then 10.25 g (49.0 mmol, 0.98 equiv) of the alkyl bromide, methyl 6-bromohexanoate, was added. The mixture was stirred at reflux for 3 hours. an additional 5.80 g (27.7 mmol, 0.55 equiv) of methyl 6-bromohexanoate was added, and the mixture was stirred at reflux for 70 hours. The mixture was cooled, diluted with 150 mL of 1 N aqueous HCl and then extracted with ethyl acetate (3 x 100 mL). The organic extracts were combined, dried over magnesium sulfate, filtered and concentrated. The residue was diluted with 200 mL of methanol and then treated with 30 mL of 10 N aqueous sodium hydroxide. The mixture was stirred at room temperature for 18 hours and then concentrated. The residue was diluted with 200 mL of deionized water and acidified to pH 1-2 with 37% concentrated HCl. The solution was washed with diethyl ether (3 x 100 mL). The aqueous phase was concentrated. The residue was diluted with 200 mL of methanol and reconcentrated. The subsequent residue was diluted with 250 mL of methanol, and HCl gas was bubbled through for 2-3 minutes followed by stirring at room temperature for 3 hours. The mixture was concentrated. The residue was diluted with 300 mL of methanol and filtered to remove inorganic salts. The filtrate was treated with 3 g of activated charcoal, filtered through Celite (manufactured by J.T. Baker) and concentrated. The residue, an off-white solid, was recrystallized from 100 mL of 2-propanol to afford 7.0 g of the product as a white solid. Concentration of the filtrate and further recrystallization of the residue yielded an additional 1.65 g of the product for a total of 8.65 g (56%): ¹H-NMR (200MHz, d₆-DMSO); 3.57 (s, 3H), 2.90-2.73 (m, 4H), 2.30 (t, 4H), 1.67-1.44 (m, 8H), and 1.37-1.20 (m, 4H) ppm.

Methyl 4-methylaminobutyrate hydrochloride. To a 1 L round bottom flask, charged with 30.0 g (195 mmol) of 4-methylaminobutyric acid and 500 mL of methanol, was bubbled HCl gas for 1-2 minutes. The mixture was stirred at room temperature for 3-4 hours and then concentrated to afford 32.5 g of the product as a foamy, off-white solid (99%): ¹H-NMR (200 MHz, CD₃OD); 3.67 (s, 3H), 3.03 (t, 2H), 2.68 (s, 3H), 2.48 (t, 2H), and 2.03-1.87 (pentet, 2H) ppm.

4-Methylaminobutanol. To a 1 L round bottom flask, charged with 32.5 g (194 mmol) of the ester, methyl 4-methylaminobutyrate hydrochloride, was added 500 mL of 1 M borane in tetrahydrofuran over a 1 hour period at 0°C. After the addition was complete, the mixture was refluxed for 20 hours, cooled to 0°C, and. the excess borane was destroyed by careful addition of 100 mL of methanol. After all the methanol was added, the mixture was stirred at room temperature for 1 hour and then concentrated. The residue was diluted with 400 mL of methanol and then HCl gas was bubbled into the solution for 5 minutes. The mixture was refluxed for 16 hours. The mixture was cooled, concentrated and then diluted with 250 mL of deionized water. the product was initially free based by addition of 10 N aqueous sodium hydroxide, to a pH of 9-9.5, and then by addition of 70 g of AG 1 X-8 anion exchange resin (hydroxide form) commercially available from BioRad), and by stirring the solution for 2 hours. The resin was filtered off and washed with 150 mL of deionized water. The aqueous filtrates were combined and concentrated. The residue was diluted with 200 mL of 2-propanol and filtered. The collected solids were rinsed with 100 mL of 2-propanol. The organic filtrates were combined and concentrated. The residue was distilled under reduced pressure to afford 12.85 g of the product as a colorless oil (bp 68°C at 0.1-0.2 mm HG; 64%): ¹H-NMR (200 MHz, D₂O); 3.52 (t, 2H) , 2.56 (t, 2H), 2.31 (s, 3H), and 1.65-1.43 (m, 4H) ppm.

4-(N-Methyl-trifluoroacetamido)-1-butanol. To a 250 mL round bottom flask, charged with 10.0 g (96.9 mmol) of the amine, 4-methylaminobutanol, in 100 mL of dry methanol, was added 17.5 mL (147 mmol) of ethyl trifluoroacetate. The mixture was stirred at room temperature for 24 hours and then concentrated to afford 18.55 g of the product as a near colorless oil (96%): ¹H-NMR (200 MHz, D₂O); 3.63 and 3.50 (2t's, 4H), 3.20 and 3.05 (d and s, 3H), and 1.82-1.47 (m, 4H) ppm.

1-(p-Toluenesulfonyloxy)-4-(N-methyltrifluoroacetamido)butane. To a 1 L dry round bottom flask, charged with 17.0 g (85.4 mmol) of the alcohol, 4-(N-methyl-trifluoroacetamido-1-butanol, in,400 mL of methylene chloride, was added 17.1 g (89.7 mmol, 1.05 equiv) of toluenesulfonyl chloride followed by 30 mL (213 mmol, 2.5 equiv) of triethylamine at 0°C over a 10 minute period. The mixture was stirred at 0°C to room temperature for 15 hours and then washed with 5% v/v aqueous HCl (3 x 200 mL). the organic phase was dried over magnesium sulfate, filtered and concentrated. The residue was chromatographed on silica gel, eluting with 50:50 hexane/methylene chloride and then with methylene chloride, to give 25.1 g of the product as a pale yellow oil (83%): ¹H-NMR (200 MHz, CDCL₃); 7.80 (d, 2H), 7.37 (d, 2H), 4.07 (m, 2H), 3.41 (m, 3H), 3.09 and 2.98 (q and s, 3H), 2.45 (s, 3H), and 1.68 (m, 4H) ppm: TLC (methylene chloride) R_{f} = 0.31.

1-S-(2,3,4,6-tetra-O-acetyl-beta-D-galactopyranosyl)-2-thiopseudourea hydrobromide. To a 250 mL round bottom flask, charged with 5.08 g (60.8 mmol, 1.09 equiv) of thiourea and 36 mL of acetone, was added 25.0 g (66.7 mmo91) of tetra-acetyl-alpha-D-galactopyranosyl bromide. The mixture was stirred at reflux for 15-20 minutes and then cooled on ice. The mixture was filtered into a Buchner funnel and rinsed with 25 mL of ice cold acetone. The solids were treated with 50 mL of acetone, refluxed for 15 minutes, cooled on ice, and filtered. The solids were rinsed with 25 mL of cold acetone, air dried and then dried under vacuum to give 22.6 g of the product as a white solid (76%): ¹H-NMR (200MHz, d₆-DMSO); 9.4-9.0 (broad d, 4H), 5.63 (d, 1H), 5.38 (d, 1H), 5.23 (dd, 1H), 5.09 (t, 1H), 4.40 (t, 1H), 4.04 (dd, 1H), 2.13 (s, 3H), 2.08 (s, 3H), 2.00 (s, 3H), 1.93 (s, 3H) ppm.

4-(N-Methylaminobutyl)-1-thio-beta-D-galactopyranoside. To a 500 mL round bottom flask, charged with 20.7 g (42.5 mmol, 1.07 equiv) of the thiopseudourea hydrobromide prepared as described above in 70 mL of deionized water, was added 6.4 g (46.3 mmol, 1.16 equiv) of potassium carbonate and 4.7 g (45.2 mmol, 1.13 equiv) of sodium bisulfite followed immediately by 14.1 g (39.9 mmol, 1.0 equiv) of the tosylate, 1-(p-toluenesulfonyloxy)-4-(N-methyltrifluoroacetamido)butane, in 70 mL of acetone. The mixture was stirred at room temperature for 16 hours. The mixture was diluted with 50 mL of brine and extracted with ethyl acetate (3 x 200 mL). The organic extracts were combined, dried over magnesium sulfate, filtered and concentrated. The residue was chromatographed on silica gel, eluting first with 75% methylene chloride/hexane, followed by methylene chloride, then with 2% methanol/methylene chloride and finally with 10% methanol/methylene chloride. Fractions containing alkylation product with different degrees of acetylation were combined and concentrated. The residue was diluted with 250 mL of methanol and 150 mL of deionized water and treated with 110 g of AG-1 X-8 resin (hydroxide form; 2.6 m equiv/g dry weight) commercially available from BioRad. The mixture was stirred at room temperature for 18 hours. The mixture was filtered, and the resin was rinsed with methanol (2 x 150 mL). The filtrates were combined and concentrated to afford 6.1 g of product (54%) : ¹H-NMR (200 MHz, D₂O); 4.38 (d, 1H), 3.88 (d, 1H), 3.69-3.41 (m, 5H), 2.82-2.64 (m, 4H), 2.43 (s, 3H), and 1.68-1.57 (, 4H) ppm.

N-BOC-Bis-methylester. To 1.00 g (3.23 mmol) of the amine hydrochloride, N,N-bis-(6-methoxycarbonylhexyl)amine hydrochloride prepared as described above, was added 1.5 mL (10.6 mmol) of triethylamine followed by 875 mg (3.55 mmol, 1.1 equiv) of BOC-ON, 2-(tert-butoxycarbonyloxyimino)-2-phenylacetonitrile. The mixture was stirred at room temperature for 18 hours and then concentrated. The residue was diluted with 100 mL of ethyl acetate and washed with 1 N aqueous hydrochloric acid (3 x 50 mL), followed by saturated aqueous sodium bicarbonate (2 x 50 mL). The organic, phase was dried over magnesium sulfate, filtered and concentrated. the residue was chromatographed on silica gel, eluting with 15% (percentage by volume) ethyl acetate/hexane. Chromatographic fractions containing product were combined and concentrated to afford 990 mg of product as a near colorless oil (83%).

N-BOC-Bis-acid. To 980 mg (2.62 mmol) of the diester prepared in the previous step in 10 mL of methanol was added 5.8 mL of 1 N aqueous sodium hydroxide (5.8 mmol). The mixture was stirred at room temperature for 16 hours and then concentrated. The residue was diluted with 30 mL of deionized water and acidified to pH 1.5-2. The mixture was extracted with ethyl acetate (6 x 50 mL). The organic extracts were dried over magnesium sulfate, filtered and concentrated. The residue was chromatographed on reverse phase C-18 silica gel commercially available from J.T. Baker, eluting with 65% methanol/water. Chromatographic fractions containing product were combined and concentrated to afford 851 mg of product as a near colorless oil (94%).

N-BOC-Tetra-methyl ester. To 825 mg (2.39 mmol) of the bis-acid prepared as described above in 35 mL of dry dimethylformamide, was added 1.75 g (5.65 mmol, 2.36 equiv) of amine hydrochloride, N,N-bis-(6-methoxycarbonylhexyl)amine hydrochloride, and 3.0 mL of triethylamine followed by 2.4 g (5.4 mmol, 2.3 equiv) of BOP. The mixture was stirred at room temperature for 17 hours and then concentrated. The residue was diluted with 100 mL of ethyl acetate and washed with 1 N hydrochloric acid (3 x 50 mL) followed by washing with aqueous sodium bicarbonate (2 x 50 mL). The organic phase was dried over magnesium sulfate, filtered and concentrated. The residue was chromatographed on silica gel, eluting with ethyl acetate. Chromatographic fractions containing product were combined and concentrated to afford 1.63 g of the product as a near colorless oil (80%).

N-BOC-Tetra-acid. To a solution of 1.41 g (1.65 mmol) of tetra-methyl ester prepared as described above in 25 mL of methanol was added 7.4 mL (7.4 mmol) of 1 N aqueous sodium hydroxide. The mixture was stirred at room temperature for 22 hours and then concentrated. The residue was diluted with 30 mL of deionized water and acidified to pH 2 with 1 N aqueous hydrochloric acid. The mixture was extracted with 3:1 (ratio by volume) ethyl acetate/isopropanol (3 x 100 mL). The organic extracts were concentrated. The residue was chromatographed on reverse phase C-18 silica gel, eluting initially with 50:50 (ratio by volume) methanol/water and eventually with 75:25 methanol/water. Chromatographic fractions containing product were combined and concentrated to afford 1.19 g of the product as a colorless oil (90%).

N-BOC Octa-methyl ester. To a mixture of 501 mg (0.626 mmol) of tetra-acid prepared as described above and 30 mL of dry dimethylformamide was added 968 mg (3.12 mmol, 5.0 equiv) of amine hydrochloride, N,N'-bis-(6-methoxycarboxyhexyl)amine hydrochloride, and 2.0 mL (14.2 mmol) of triethylamine, followed by 1.22 g (2.76 mmol, 4.6 equiv) BOP. The mixture was stirred at room temperature for 19 hours and then concentrated. The residue was diluted with 75 mL of ethyl acetate and washed with 1 N aqueous hydrochloric acid (2 x 50 mL). The organic phase was dried over magnesium sulfate, filtered and concentrated. The residue was chromatographed on reverse phase C-18 silica gel, eluting initially with 60:40 methanol/water and eventually with 90:10 methanol/water. The chromatographic fractions containing product were combined and concentrated to afford 715 mg of the product as a colorless oil (63%).

N-BOC Octa-acid. To a solution of 715 mg (0.393 mmol) of octa-methyl ester prepared as described above in 20 mL of methanol was added 6 mL of 1 N aqueous , sodium hydroxide (6 mmol) and 5 mL of deionized water. The mixture was stirred at room temperature for 16 hours and then concentrated. The residue was diluted with 20 mL of deionized water, and the solution was acidified to pH 1.5-2.0. The mixture was concentrated, and the residue was chromatographed on reverse phase C-18 silica gel, eluting initially with 50:50 methanol/water and eventually with 80:20 methanol/water. The chromatographic fractions containing product were combined and concentrated to afford 647 mg of the product as a near colorless oil (96%).

The above procedure is designed for the formation of a galactose cluster of 8 galactose residues. The four galactose version could be made in accordance with this procedure by proceeding from the tetra acid to the galactose derivatization step, which is described below for the 8-galactose cluster. Similarly, 16, 32, etc. galactose cluster constructs can be prepared in accordance with the present invention by introduction of more iterations of the methyl ester and acid formation steps. More specifically, the 16-methyl ester construct, the 16-acid, the 32-methyl ester and so on would be prepared essentially as described above for the tetra and octa forms. When the desired number of acid residues are formed, the galactose derivatization step is employed, with the proportions of the components adjusted to accommodate the number of acid residues.

N-BOC-Octa-galactosyl construct. To a mixture of 161 mg (94 mmol) of octa-acid prepared as described above and 225 mg (906 micromol, 9.64 equiv) of galactose amine, 4-N-methylaminobutyl)-1-thio-beta-D-galactopyranoside, in 8 mL of dry dimethylformamide was added 0.5 mL (3.54 mmol) of triethylamine followed by 371 mg (839 micromol, 8.4 equiv) of BOP. The mixture was stirred at room temperature for 17 hours and then concentrated. The residue was chromatographed on reverse phase C-18 silica gel, eluting initially with 40:60 methanol/water and finally with 70:30 methanol/water. The chromatographic fractions containing product were combined and concentrated to afford 170 mg of the product as a near colorless oil (47%).

Octa-galactosyl amine. To 170 mg of the N-BOC-octa-galactosyl construct prepared as described above was added 5 mL of trifluoroacetic acid. The mixture was stirred at room temperature for 10 minutes and then concentrated. The residue was diluted with 10 mL of methanol and reconcentrated. The residue is used without further purification.

### B. Extender-Galactose Cluster Preparation.

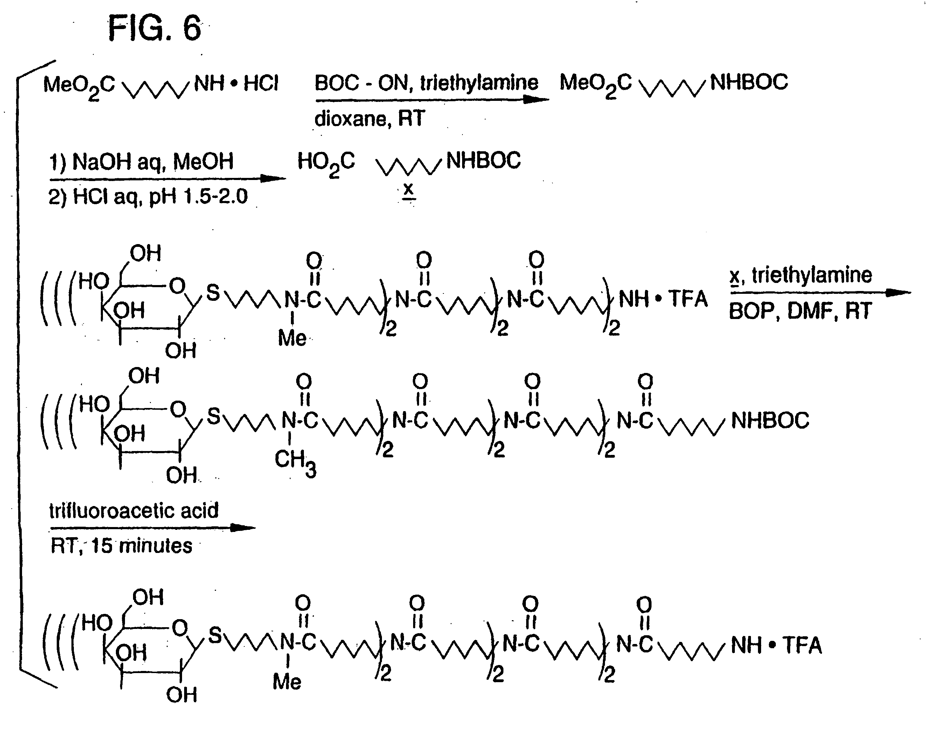

This procedure is schematically shown in Fig. 6. Methyl 6-(N-BOC)-aminocaproate. To a mixture of amine hydrochloride, methyl-6-aminohexanoate hydrochloride, prepared as described above is added 1.1 equivalents of BOC-ON followed by 2-3 equivalents of triethylamine. The mixture is stirred at 15-30°C for 16-24 hours and the concentrated. The residue is dissolved in ethyl acetate and washed with 1 N aqueous hydrochloric acid and then with saturated aqueous sodium bicarbonate. The organic phase is dried over magnesium sulfate, filtered and concentrated via reduced pressure rotary evaporation. The residue is chromatographed on silica gel, eluting with 25% ethyl acetate/hexane. The chromatographic fractions containing the product are combined and concentrated to afford the product.

6-(N-BOC)-aminocaproic acid. To a solution of the methyl ester, methyl 6-(N-BOC)-aminocaproate, in methanol is added 1.5 equivalents of 1 N aqueous sodium hydroxide. The mixture is stirred at 15-30°C for 16-24 hours and then concentrated. The residue is diluted with deionized water and extracted with ethyl acetate. The organic extracts are combined, dried over magnesium sulfate, filtered and concentrated. The residue is chromatographed on silica gel, eluting initially with 25% ethyl acetate/hexane and finally with 100% ethyl acetate. The chromatographic fractions containing the product are combined and concentrated to afford the product.

N-BOC extended octa-galactosyl construct. To a solution of the octa-galactosyl amine prepared as described above in dimethylformamide and 1.5-3 equivalents of 6-(N-BOC)-aminocaproic acid is added 4-6 equivalents of triethylamine followed by 1.1-1.5 equivalents of BOP. The mixture is stirred at 15-30°C for 4-24 hours and then concentrated. The residue is diluted with deionized water, and the pH is adjusted. to 1.5-2.0 by addition of 1 N aqueous hydrochloric acid. The mixture is washed with ethyl acetate. The aqueous phase is concentrated, and the residue is chromatographed on reverse phase C-18 silica gel, eluting initially with 50:50 methanol/water and finally with 65:35 methanol/water. The chromatographic fractions containing product are combined and concentrated to afford the product.

Amine extended octa-galactosyl construct. To the N-BOC protected amine prepared in the previous step is added trifluoroacetic acid. The mixture is stirred at 15-30°C for 10 minutes and then concentrated. The residue is diluted with methanol and reconcentrated to afford the product which is used without further purification.

### C. Conjugation of Galactose Cluster with Chelate and Annexin Components.

Octa-galactosyl-chelate construct. To a mixture of amine extended octa-galactosyl amine, 1.2 equivalents of N,N'-bis(2-disulfidyl-4-hydroxycarbonylphenyl)-1,3-propyldiamine mono-(methylaminocaproate) adduct, and 5 equivalents of triethylamine in dimethylformamide is added 1.1 equivalents of BOP. The mixture is stirred at 15-30°C for 2 to 24 hours and then concentrated. The residue is diluted with deionized water, and the pH is adjusted to 2.5 by the addition of 1 N aqueous hydrochloric acid. The mixture is washed with ethyl acetate. The aqueous phase is concentrated. The residue is chromatographed on reverse phase C-18 silica gel. The fractions containing the product are combined and concentrated to give the product.

Octa-galactosyl-chelate carboxylic acid construct. To a methanolic solution of the ester bearing octagalatosyl-chelate construct prepared previously is added 4-5 equivalents of 1 N aqueous sodium hydroxide. The mixture is stirred at 15-30°C for 16-24 hours. The mixture is concentrated, and the residue is diluted with deionized water. The pH of the resulting solution is adjusted to approximately 2.5, and the solution is reconcentrated. The residue is chromatographed on reverse phase C-18 silica gel. The fractions containing the product are combined and concentrated to afford the product.

Annexin V-chelate-octa-galactosyl construct. The octa-galactosyl-chelate carboxylic acid construct is offered to annexin V in molar ratios of 30:1, 15:1, 5:1 and 2:1. The conjugation of annexin V is carried out via activation of the carboxylic acid functional group of the galactose cluster-chelate construct with benzotriazol-1-yl-oxy-tris-(dimethylamino)phosphonium hexafluorophosphate (BOP). To the galactose cluster-chelate construct in DMF, an equimolar concentration of BOP in DMF is added. Annexin V in 0.05 M HEPES buffer (commercially available from Sigma Chemical Co., St. Louis, Missouri), pH 7.4 is added to the reaction mixture followed by addition of 0.5 M borate buffer, pH 8.5. The DMF concentration in the reaction mixture is maintained between 5-20%. The solution is mixed for 1 hour at room temperature by tumbling the reaction vessel, and is then allowed to stand at room temperature overnight (approximately 18-24 hours). The reaction mixture is then diluted with PBS, transferred to dialysis tubing (6000-8000 molecular weight cut off), and dialyzed for 24 hours against PBS. After removing the material from the dialysis bag, the solution is filtered through a 0.2 micrometer syringe filter. Ail of the offering levels are conducted analogously.

### D. Characterization of Galactose Cluster-Chelate-Annexin V Conjugate.

Protein concentration is determined using A₂₈₀ of 0.6 for a 1 mg/mL solution of annexin V. The number of galactose residues per molecule of annexin V is determined by measuring the total number of reactive amines on Annexin V before and after reaction with galactosyl cluster-chelate conjugate using trinitrobenzenesulfonic acid, as described by Habeeb, Analytical Biochemistry, 14: 328-36, 1966. The ability of galactose-chelate-annexin V to bind to activated platelets is assessed by determining its ability to inhibit the binding of unmodified, I-125-radiolabeled annexin V to freshly isolated human platelets, following the method of Thiagarajan and Tait, J. Biol. Chem., 265: 17,240-43, 1990.

### E. Radiolabeling Procedure for use in Pre-Formed Chelate Conjugation Method.

Method A: Stannous gluconate kits are prepared containing 5 mg sodium gluconate, 100 micrograms stannous chloride, 1.0 mg (1 mg/mL) of galactose cluster-chelate-annexin V, and 0.1 to 1.0 mg of lactose. The pH is maintained between 5 and 7 using HCl, acetic acid or NaOH. To the stannous gluconate kit is added 1.0mL sodium pertechnetate (Tc-99m) with a specific activity of about 50 mCi. The vial is incubated at 25-37°C for 5-30 minutes. The percent formation of labeled conjugate, remaining TcO₄, and hydrolyzed reduced technetium is determined by ITLC in 12% TCA as developing solvent.

Method B: Stannous tartrate kits are prepared in an evacuator vial under nitrogen to contain 0.5 mL of disodium tartrate (10 mg/mL) and 0.1 mL stannous chloride (1.0 mg/mL in ethanol). The pH of the solution is kept between 5 and 7, preferably 6.0. To this stannous tartrate solution is added 1.0 mL of sodium pertechnetate (50 mCi), and the solution is allowed to stand at room temperature. In and evacuated vial, 200 microliters of sodium phosphate (0.5 M, pH 8 or 10) and 1.0 mL of galactose cluster-chelate-annexin V conjugate (1.0 mg/mL) are added successively. Then Tc-99m-tartrate (50 mCi) is added, and the vial is incubated at 25-37°C for 5-30 minutes. The percent formation of labeled conjugate, remaining TcO₄, and hydrolyzed reduced technetium is determined by ITLC in 12% (w/v) trichloroacetic acid as developing solvent.

Constructs prepared in accordance with this Example are tested in accordance with the procedures set forth above (Example III and Example IV(E)) to verify usefulness in clot imaging applications, for example.

### EXAMPLE VII

### Annexin-Galactose Cluster Conjugates Trifunctional Linker Approach

### A. Chelate Preparation.

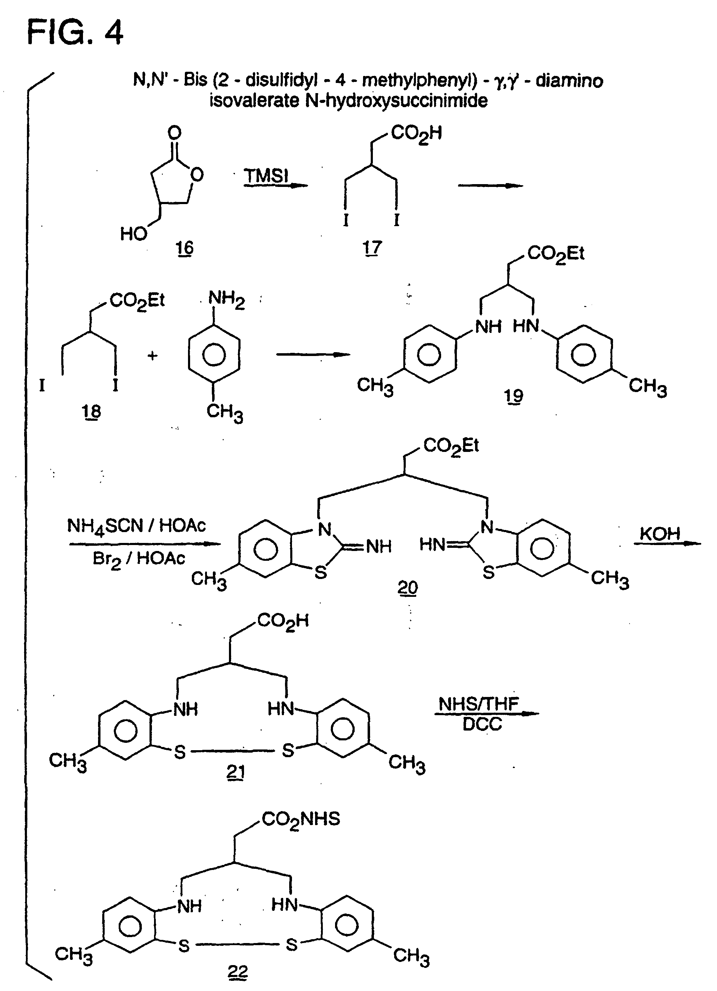

Production of chelate N,N'-bis(2-disulfidyl-4-methylphenyl)-gamma,gamma'-diamino-isovalerate N-hydroxysuccinimide, as shown schematically in Figure 4.

3-Iodomethyl-4-iodobutyric acid: To a solution of 1.61 g (10 mmole) 3-hydroxymethyl-4-butanolide (prepared by the procedure of Kinoshita and Hirano, J. Hetrocyclic Chem., 29: 1025, 1992) in 100 mL carbon tetrachloride is added 8 g (40 mmole) of iodotrimethylsilane. The reaction mixture is heated at 50°C for 12 hours under nitrogen. The mixture is diluted with chloroform and washed with water (3 x 100 mL), 5% aqueous sodium thiosulfate (100mL), 10% aqueous sodium bicarbonate and brine. The organic layer is dried over magnesium sulfate, filtered and evaporated to give the desired crude product. The crude product is purified by silica gel chromatography (ethyl acetate-hexane = 3:7 as the eluting solvent) to give 3-iodomethyl-4-iodobutyric acid.

Ethyl-3-iodomethyl-4-iodobutyrate: A solution of 2.831 g (8 mmole) 3-iodomethyl-4-iodobutyric acid in 80 mL ethanol is saturated with HCl gas at 0°C. After stirring the solution at room temperature for two days, the solvent is removed under vacuum, and the residue is dissolved in dichloromethane. The dichloromethane layer is washed with 10% aqueous sodium bicarbonate (3 x 100 mL), water (1 x 100 mL) and brine. The separated dichloromethane layer is dried over with magnesium sulfate, filtered and evaporated to give ethyl-3-iodomethyl-4-iodobutyrate.

Ethyl-gamma, gamma'-di(4-methylanilino) isovalerate: A stirred solution of 7.5 g (70 mmole) 4-toluidine, 2.764 g (7 mmole) ethyl-3-iodomethyl-4-iodobutyrate and 0.588 g (7 mmole) sodium bicarbonate in 30 mL dry dimethyl sulfoxide is heated at 100°C for 3 hours under nitrogen. The cooled mixture is poured onto 400 mL ice water with stirring. The resulting precipitate is collected by filtration. The remaining 4-toluidine in the precipitate is removed by washing with aqueous acetic acid several times. The product is obtained by recrystallization of the washed precipitate in heptane.

Ethyl-gamma,gamma'-[1,3-di(2-imino-6-methyl benzthiazolyl-3)]isovalerate: To a magnetically stirred suspension of 2.0 g (6.5 mmole) ethyl-gamma,gamma'-di(4-methylanilino)isovalerate in 250 mL glacial acetic acid is added ammonium thiocyanate (3.5 g, 0.046 mole) followed by the dropwise addition of a solution of bromine (7.27 g, 0.046 mole) in 50 mL glacial acetic acid. After addition is complete, stirring is continued overnight. The yellow precipitate of dihydrobromide salt is filtered and dried. The dried solid is then dissolved in hot water and the benzothiazole free base is liberated with saturated sodium bicarbonate solution. The white solid is filtered and dried to give crude product which is used without further purification.

N,N'-Bis(2-disulfidyl-4-methylphenyl)-gamma, gamma'-diaminoisovaleric aid: To a suspension of ethyl-gamma,gamma'-[1,3-di(2-imino-6-methyl benzthiazolyl-3)]isovalerate in 40 mL distilled water, solid potassium hydroxide pellets (20.0 g, 0.037 mole), are added, and the resulting solution is heated at 120°C for 15-24 hours. After several hours of heating, the suspension becomes a clear solution. The reaction mixture is cooled in an ice bath and acidified with 5.0 N acetic acid to pH 5.0, and the aqueous solution is extracted with three 100 mL portions of ethyl acetate. The combined ethyl acetate extracts are dried over anhydrous sodium sulfate and filtered. Solvent from the filtrate is removed under reduced pressure to give crude product. This crude product is chromatographed on silica gel column using a 20:80 mixture of ethyl acetate:hexane with 1% acetic acid as eluting solvent to give the product as a crystalline yellow solid.

N,N'-Bis(2-disulfidyl-4-methylphenyl)-gamma, gamma'-diaminoisovalerate N-hydroxysuccinimide: N,N'-Bis(2-disulfidyl-4-methylphenyl)-gamma,gamma'-diaminoisovaleric acid is reacted with N-hydroxysuccinimide (NHS) and dicyclohexylcarbodiimide (DCC) in either tetrahydrofuran (THF) or dimethylformamide (DMF) at room temperature. After stirring overnight at room temperature, the solvent is removed, and the crude product is purified by column chromatography on silica gel.

### B. Conjugate formation.

This chelate is amenable to use with a suitable trifunctional linker to form a radiolabeled annexin-galactose cluster conjugate of the present invention as described below.

Commercially available N-epsilon-t-BOC-lysine (Sigma Chemical Company) is converted, using trifluoroacetic anhydride, to its N-alphatrifluoroacetamide adduct. Activation of the carboxylic acid functionality, for example with BOP (benzotriazol-1-yloxy-tris(dimethyl-amino)-phosphonium hexafluorophosphate) commercially available from Aldrich Chemical Co., and reaction of the activated moiety with the single available amine on a galactose cluster, e.g., formed as described above, affords a galactose cluster-trifunctional linker species. The alpha-amine of lysine trifunctional linker component of the galactose cluster-trifunctional linker species is deblocked using methanolic sodium hydroxide. Reaction with the N-hydroxysuccinimide ester of the chelate molecule formed as set forth in part A of this example affords a galactose cluster-chelatetrifunctional linker species. Deprotection of the epsilon amine of the lysine trifunctional linker component using trifluoroacetic acid, followed by reaction with succinic anhydride provides an available carboxylic acid functionality through which the annexin may be conjugated following activation of the carboxylic acid (e.g., with BOP).

## Claims

1. An annexin conjugate suitable for radiolabeling with a diagnostic imaging agent comprising:
an annexin;
an N₂S₂ or N₃S compound capable of complexing a radionuclide, wherein the compound is associated with the annexin; and
a cluster of hexose or hexose based residues connected in a branched configuration, wherein the residues of said cluster are configured to be recognised by a liver receptor.

2. The conjugate of claim 1, wherein the annexin is annexin V.

3. The conjugate of claims 1 or 2, wherein the cluster is recognized by a mammalian liver receptor.

4. The conjugate of claims 1 or 2, wherein the cluster is recognized by an Ashwell receptor.

5. The conjugate of claims 1 or 2, wherein the cluster is attached at a single point to the annexin or the conjugate.

6. The conjugate of any one of claims 1-5, wherein at least one residue of the cluster is selected from galactose, mannose, mannose 6-phosphate, N-acetylglucosamine, pentamannosyl phosphate, glucose, N-galactosamine, N-acetylgalactoseamine, thioglycosides of galactose, D-galactosides and glucosides.

7. The conjugate of claim 6, wherein the cluster comprises at least three galactose residues.

8. The conjugate of any one of claims 1-5, wherein the compound is a N₂S₂ compound capable of complexing a radionuclide.

9. The conjugate of claim 8, wherein the N₂S₂ compound is selected from N,N'-Bis(2-disulfidyl-4-ethoxycarbonylphenyl)-1,3-propyldiamine, and N,N'-Bis(2-disulfidyl-4-methylphenyl)-γ,γ'-diaminoisovalerate.

10. The conjugate of claim 8, wherein the N₂S₂ compound is N, N'-Bis(2-disulfidyl-4-ethoxycarbonylphenyl)-1,3-propyldiamine.

11. The conjugate of claim 8, wherein the N₂S₂ compound is N,N'-Bis(2-disulfidyl-4-methylphenyl)-γ,γ'-diaminoisovalerate.

12. The compound of any one of claims 1-5, wherein the compound is a N₃S compound capable of complexing a radionuclide.

13. The conjugate of claim 12, wherein the N₃S compound is S-benzoylmercaptoacetyl-glycylglycylglycine.

14. The conjugate of any one of claims 1-5, wherein the annexin is modified by the addition of from 2 to 6 terminal amino acid residues.

15. The conjugate of any one of claims 1-5, wherein the compound is of the following structure: 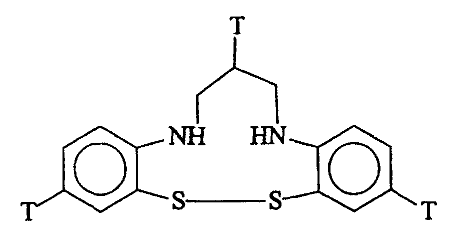 wherein T bears a functional group useful for conjugation.

16. The conjugate of claim 15, wherein the conjugate is configured as follows: hexose cluster - chelate - annexin, and the components are covalently connected by bifunctional linkers.

17. The conjugate of any one of claims 1-5, wherein the branched configuration of the cluster comprises a linker moiety attached to one or more of the hexose residues.

18. The conjugate of claim 5, wherein the single point of attachment is via a linker moiety from the hexose cluster to the annexin.

19. The conjugate of any one of claims 1-5, wherein the attachment of the cluster is to a linker moiety which is part of the annexin or the compound.

20. The conjugate of any one of claims 1-5, wherein the hexose cluster is a galactose cluster.

21. The conjugate of claim 20, wherein the galactose cluster is of the following formula: 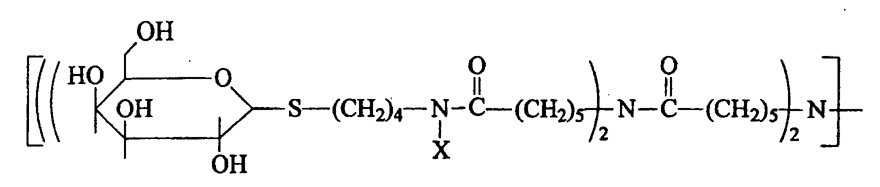 wherein X is H or methyl.

22. The conjugate of claim 20, wherein the galactose cluster is of the following formula: 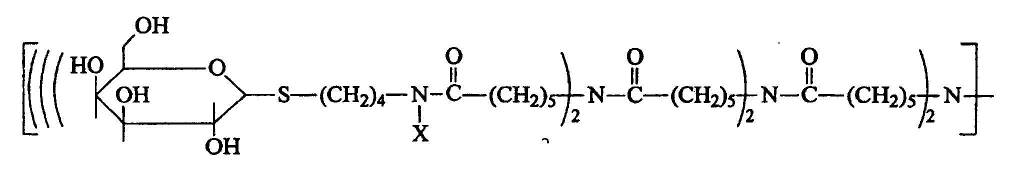 wherein X is H or methyl.

23. The conjugate of claim 20, wherein the galactose cluster is of the following formula: 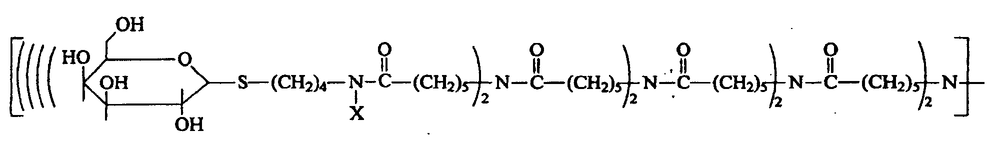 wherein X is H or methyl.

24. The conjugate of claim 20, wherein the galactose cluster is of the following formula: 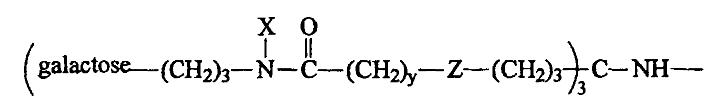 wherein X is H or methyl, y is from 1-10, and Z is O or S.

25. The conjugate of claim 20, wherein the galactose cluster is of the following formula: 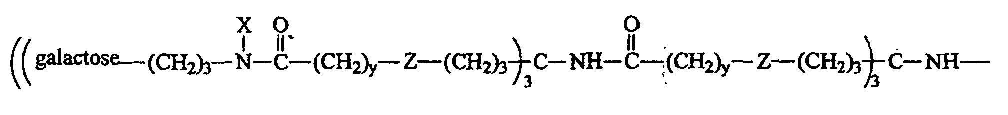 wherein X is H or methyl, y is from 1-10, and Z is O or S.

26. The conjugate of claim 20, wherein the galactose cluster is of the following formula: 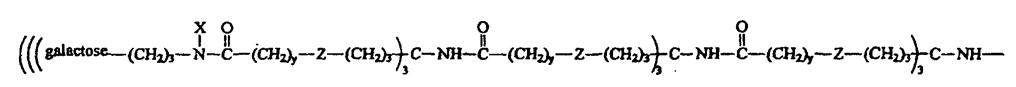 wherein X is H or methyl, y is from 1-10, and Z is O or S.

27. The conjugate of any one of claims 1-26, wherein a radionuclide is complexed by the compound.

28. The conjugate of claim 27, wherein the radionuclide is selected from the group consisting essentially of Cu-64, Re-186, Re-188, Pd-100, Bi-212, Pb-212, Pd-109, Cu-67, Tc-99m, Tc-94, Ru-95, Ru-105, Rh-99, Rh-105, In-111, Sm-153, Lu-177, Lu-170, Pt-189, Pt-193, Au-199 and Hg-197.

29. The conjugate of claim 27, wherein the radionuclide is Tc-99m.

30. The conjugate of Claim 8 when dependent upon Claim 2 wherein the N₂S₂ compound is 4,5-bis(S-1-ethoxyethylmercapto-acetamido)pentanoate, and wherein the hexose cluster is a galactose cluster.

## Patentansprüche

1. , Annexin-Konjugat, das zur radioaktiven Markierung mit einem Mittel zur diagnostischen Bildgebung geeignet ist, welches umfaßt:
ein Annexin;
eine N₂S₂- oder N₃S-Verbindung, die ein Radionuklid komplexieren kann, wobei die Verbindung mit dem Annexin assoziiert ist; und
einen Cluster aus Hexoseresten oder Resten auf Hexosebasis, die in einer verzweigten Konfiguration verknüpft sind, wobei die Reste besagten Clusters so konfiguriert sind, daß sie von einem Leberrezeptor erkannt werden.

2. Konjugat nach Anspruch 1, **dadurch gekennzeichnet, daß** das Annexin Annexin V ist.

3. Konjugat nach den Ansprüchen 1 oder 2, **dadurch gekennzeichnet, daß** der Cluster von einem Säuger-Leberrezeptor erkannt wird.

4. Konjugat nach den Ansprüchen 1 oder 2, **dadurch gekennzeichnet, daß** der Cluster von einem Ashwell-Rezeptor erkannt wird.

5. Konjugat nach den Ansprüchen 1 oder 2, **dadurch gekennzeichnet, daß** der Cluster an einem einzigen Punkt an das Annexin oder das Konjugat gebunden ist.

6. Konjugat nach einem der Ansprüche 1 bis 5, **dadurch gekennzeichnet, daß** wenigstens ein Rest des Clusters ausgewählt ist aus Galactose, Mannose, Mannose-6-phosphat, N-Acetylglucasamin, Pentamannosylphosphat, Glucose, N-Galactosamin, N-Acetylgalactosamin, Thioglykosiden von Galactose, D-Galactosiden und Glucosiden.

7. Konjugat nach Anspruch 6, **dadurch gekennzeichnet, daß** der Cluster wenigstens drei Galactosereste umfaßt.

8. Konjugat nach einem der Ansprüche 1 bis 5, **dadurch gekennzeichnet, daß** die Verbindung eine N₂S₂-Verbindung ist, die ein Radionuklid komplexieren kann.

9. Konjugat nach Anspruch 8, **dadurch gekennzeichnet, daß** die N₂S₂-Verbindung ausgewählt ist aus N,N'-Bis(2-disulfidyl-4-ethoxycarbonylphenyl)-1,3-propyldiamin und N,N'-Bis(2-disulfidyl-4-methylphenyl)-γ,γ'-diaminoisovalerat.

10. Konjugat nach Anspruch 8, **dadurch gekennzeichnet, daß** die N₂S₂-Verbindung N,N'-Bis(2-disulfidyl-4-ethoxycarbonylphenyl)-1,3-propyldiamin ist.

11. Konjugat nach Anspruch 8, **dadurch gekennzeichnet, daß** die N₂S₂-Verbindung N,N'-Bis(2-disulfidyl-4-methylphenyl)-y,y'-diaminoisovalerat ist.

12. Verbindung nach einem der Ansprüche 1 bis 5, **dadurch gekennzeichnet, daß** die Verbindung eine N₃S-Verbindung ist, die ein Radionuklid komplexieren kann.

13. Konjugat nach Anspruch 12, **dadurch gekennzeichnet, daß** die N₃S-Verbindung S-Benzoylmercaptoacetylglycylglycylglycin ist.

14. Konjugat nach einem der Ansprüche 1 bis 5, **dadurch gekennzeichnet, daß** das Annexin durch die Addition von 2 bis 6 terminalen Aminosäureresten modifiziert ist.

15. Konjugat nach einem der Ansprüche 1 bis 5, **dadurch gekennzeichnet, daß** die Verbindung die folgende Struktur aufweist: 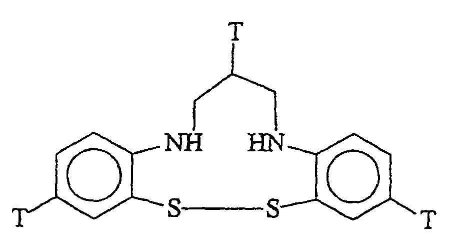 in der T eine für die Konjugation nützliche funktionelle Gruppe trägt.

16. Konjugat nach Anspruch 15, **dadurch gekennzeichnet, daß** das Konjugat wie folgt konfiguriert ist: Hexose-Cluster - Chelat - Annexin und die Komponenten kovalent durch bifunktionelle Linker verbunden sind.

17. Konjugat nach einem der Ansprüche 1 bis 5, **dadurch gekennzeichnet, daß** die verzweigte Konfiguration des Clusters eine Linkereinheit umfaßt, die an einen oder mehrere der Hexosereste gebunden ist.

18. Konjugat nach Anspruch 5, **dadurch gekennzeichnet, daß** der einzige Bindungspunkt über eine Linkereinheit vom Hexose-Cluster zum Annexin ist.

19. Konjugat nach einem der Ansprüche 1 bis 5, **dadurch gekennzeichnet, daß** die Bindung des Clusters an eine Linkereinheit ist, die Teil des Annexins oder der Verbindung ist.

20. Konjugat nach einem der Ansprüche 1 bis 5, **dadurch gekennzeichnet, daß** der Hexose-Cluster ein Galactose-Cluster ist.

21. Konjugat nach Anspruch 20, **dadurch gekennzeichnet, daß** der Galactose-Cluster die folgende Formel aufweist: 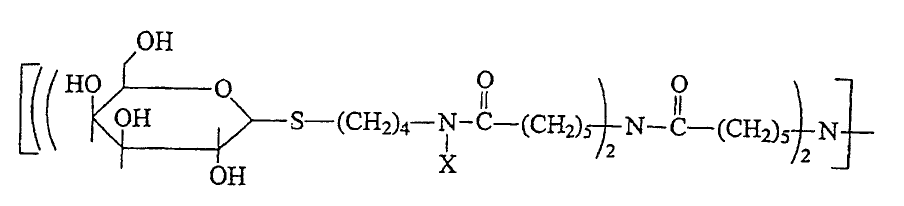 in der X H oder Methyl ist.

22. Konjugat nach Anspruch 20, **dadurch gekennzeichnet, daß** der Galactose-Cluster die folgende Formel aufweist: 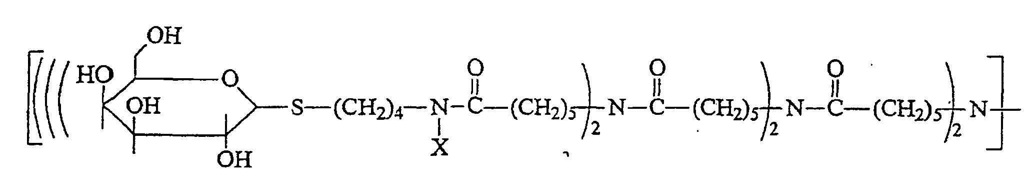 in der X H oder Methyl ist.

23. Konjugat nach Anspruch 20, **dadurch gekennzeichnet, daß** der Galactose-Cluster die folgende Formel aufweist: 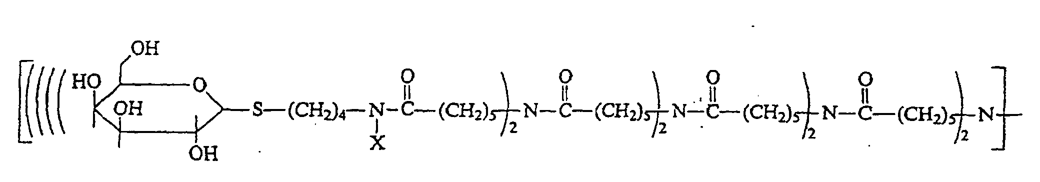 in der X H oder Methyl ist.

24. Konjugat nach Anspruch 20, **dadurch gekennzeichnet, daß** der Galactose-Cluster die folgende Formel aufweist: 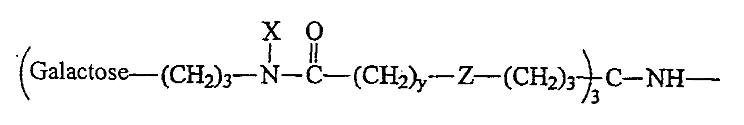 in der X H oder Methyl ist, y von 1 bis 10 ist und Z O oder S ist.

25. Konjugat nach Anspruch 20, **dadurch gekennzeichnet, daß** der Galactose-Cluster die folgende Formel aufweist: 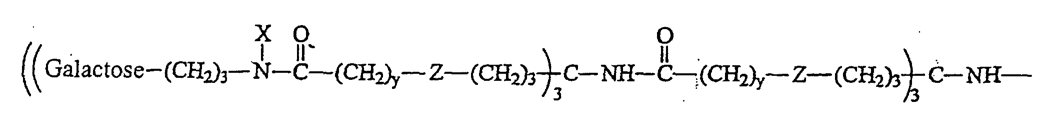 in der X H oder Methyl ist, y von 1 bis 10 ist und Z O oder S ist.

26. Konjugat nach Anspruch 20, **dadurch gekennzeichnet, daß** der Galactose-Cluster die folgende Formel aufweist: 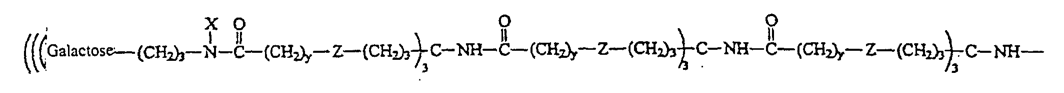 in der X H oder Methyl ist, y von 1 bis 10 ist und Z O oder S ist.

27. Konjugat nach einem der Ansprüche 1 bis 26, **dadurch gekennzeichnet, daß** ein Radionuklid von der Verbindung komplexiert ist.

28. Konjugat nach Anspruch 27, **dadurch gekennzeichnet, daß** das Radionuklid ausgewählt ist aus der Gruppe, im wesentlichen bestehend aus Cu-64, Re-186, Re-188, Pd-100, Bi-212, Pb-212, Pd-109, Cu-67, Tc-99m, Tc-94, Ru-95, Ru-105, Rh-99, Rh-105, In-111, Sm-153, Lu-177, Lu-170, Pt-189, Pt-193, Au-199 und Hg-197.

29. Konjugat nach Anspruch 27, **dadurch gekennzeichnet, daß** das Radionuklid Tc-99m ist.

30. Konjugat nach Anspruch 8, wenn abhängig von Anspruch 2, **dadurch gekennzeichnet, daß** die N₂S₂-Verbindung 4,5-Bis(S-1-ethoxyethylmercapto-acetamido)pentanoat ist und daß der Hexose-Cluster ein Galactose-Cluster ist.

## Revendications

1. Conjugué d'annexine convenant à un radiomarquage avec un agent d'imagerie de diagnostic, comprenant :
une annexine,
un composé N₂S₂ ou N₃S susceptible de complexer un radionuclide, où le composé est associé à l'annexine, et
un agglomérat de résidus hexose ou à base d'hexose relié suivant une configuration ramifiée, où les résidus dudit agglomérat sont configurés pour être reconnus par un récepteur du foie.

2. Conjugué selon la revendication 1, dans lequel l'annexine est l'annexine V.

3. Conjugué selon les revendications 1 ou 2, dans lequel l'agglomérat est reconnu par un récepteur du foie de mammifère.

4. Conjugué selon les revendications 1 ou 2, dans lequel l'agglomérat est reconnu par un récepteur d'Ashwell.

5. Conjugué selon les revendications 1 ou 2, dans lequel l'agglomérat est fixé en un seul point à l'annexine ou au conjugué.

6. Conjugué selon l'une quelconque des revendications 1 à 5, dans lequel au moins un résidu de l'agglomérat est choisi parmi le galactose, le mannose, le mannose 6―phosphate, la N―acétylglucosamine, le phosphate de pentamannosyle, le glucose, la N-galactosamine, la N-acétylgalactosamine, les thioglucosides du galactose, les D-galactosides et les glucosides.

7. Conjugué selon la revendication 6, dans lequel l'agglomérat comprend au moins trois résidus galactose.

8. Conjugué selon l'une quelconque des revendications 1 à 5, dans lequel le composé est un composé N₂S₂ susceptible de complexer un radionuclide.

9. Conjugué selon la revendication 8, dans lequel le composé N₂S₂ est choisi parmi la N,N'-bis(2-disulfidyl-4-éthoxycarbonylphényl)-1,3-propyldiamine et le γ,γ'-diaminoisovalérate de N,N'-bis(2-disulfidyl-4-méthylphényle).

10. Conjugué selon la revendication 8, dans lequel le composé N₂S₂ est la N,N'-bis(2-disulfidyl-4-éthoxycarbonylphényl)-1,3-propyldiamine.

11. Conjugué selon la revendication 8, dans lequel le composé N₂S₂ est le γ,γ'-diaminoisovalérate de N,N'-bis(2-disulfidyl-4-méthylphényle).

12. Composé selon l'une quelconque des revendications 1 à 5, dans lequel le composé est un composé N₃S susceptible de complexer un radionuclide.

13. Conjugué selon la revendication 12, dans lequel le composé N₃S est la S-benzoylmercapto-acétylglycylglycylglycine.

14. Conjugué selon l'une quelconque des revendications 1 à 5, dans lequel l'annexine est modifiée par l'addition de 2 à 6 résidus acides aminés terminaux.

15. Conjugué selon l'une quelconque des revendications 1 à 5, dans lequel le composé est de la structure suivante : 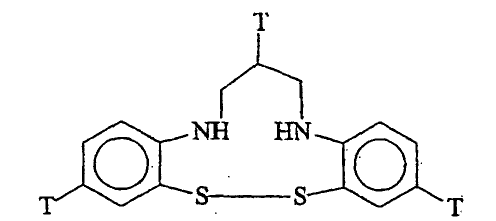 dans laquelle T porte un groupement fonctionnel utile pour la conjugaison.

16. Conjugué selon la revendication 15, dans lequel le conjugué est configuré comme suit : agglomérat d'hexoses―chélate―annexine et les composants sont liés de manière covalente par des segments de liaison bifonctionnels.

17. Conjugué selon l'une quelconque des revendications 1 à 5, dans lequel la configuration ramifiée de l'agglomérat comprend une fraction de segment de liaison fixée à un ou plusieurs des résidus hexose.

18. Conjugué selon la revendication 5, dans lequel le point unique de fixation est réalisé par l'intermédiaire d'une fraction de segment de liaison de l'agglomérat d'hexoses à l'annexine.

19. Conjugué selon l'une quelconque des revendications 1 à 5, dans lequel la fixation de l'agglomérat est réalisée sur une fraction de segment de liaison qui fait partie de l'annexine ou du composé.

20. Conjugué selon l'une quelconque des revendications 1 à 5, dans lequel l'agglomérat d'hexoses est un agglomérat de galactoses.

21. Conjugué selon la revendication 20, dans lequel l'agglomérat de galactoses est de la formule suivante : 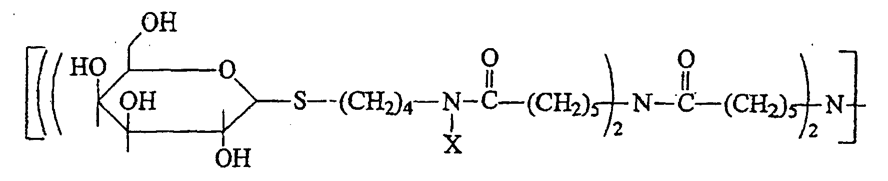 dans laquelle X est H ou un méthyle.

22. Conjugué selon la revendication 20, dans lequel l'agglomérat de galactoses est de la formule suivante : 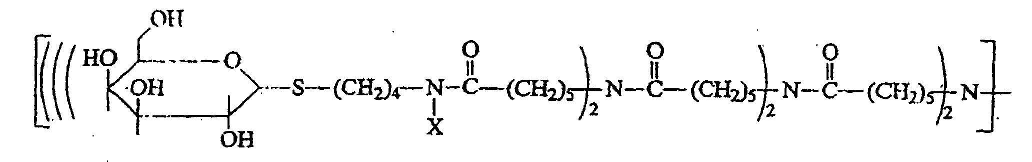 dans laquelle X est H ou un méthyle.

23. Conjugué selon la revendication 20, dans lequel l'agglomérat de galactoses est de la formule suivante : 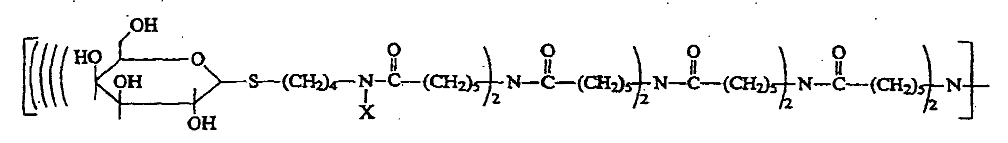 dans laquelle X est H ou un méthyle.

24. Conjugué selon la revendication 20, dans lequel l'agglomérat de galactoses est de la formule suivante : 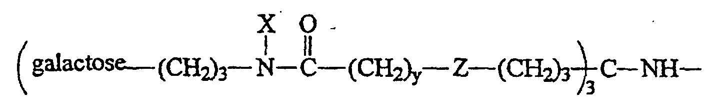 dans laquelle X est H ou un méthyle, y vaut de 1 à 10 et Z est O ou S.

25. Conjugué selon la revendication 20, dans lequel l'agglomérat de galactoses est de la formule suivante : 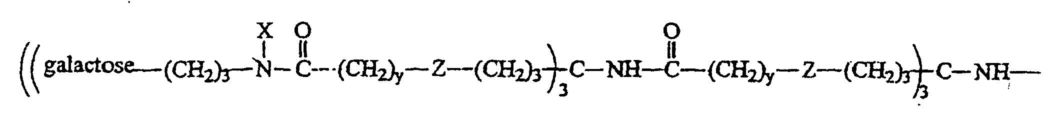 dans laquelle X est H ou un méthyle, y vaut de 1 à 10 et Z est O ou S.

26. Conjugué selon la revendication 20, dans lequel l'agglomérat de galactoses est de la formule suivante : 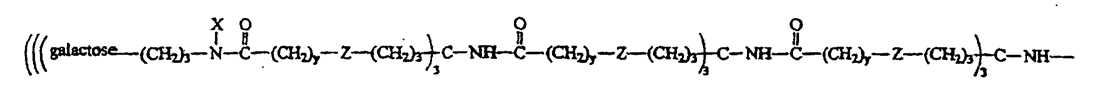 dans laquelle X est H ou un méthyle, y vaut de 1 à 10 et Z est O ou S.

27. Conjugué selon l'une quelconque des revendications 1 à 26, dans lequel un radionuclide est complexé par le composé.

28. Conjugué selon la revendication 27, dans lequel le radionuclide est choisi parmi le groupe constitué essentiellement de Cu-64, Re-186, Re-188, Pd-100, Bi-212, Pb-212, Pd-109, Cu-67, Tc-99m, Tc-94, Ru-95, Ru-105, Rh-99, Rh-105, In-111, Sm-153, Lu-177, Lu-170, Pt-189, Pt-193, Au-199 et Hg-197.

29. Conjugué selon la revendication 27, dans lequel le radionuclide est Tc-99m.

30. Conjugué selon la revendication 8, lorsqu'elle dépend de la revendication 2, dans lequel le composé N₂S₂ est le 4,5-bis(S-1-éthoxyéthylmercapto-acétamido)pentanoate et dans lequel l'agglomérat d'hexoses est un agglomérat de galactoses.
